# EUROPEAN PATENT APPLICATION

(11) **EP 4 653 460 A1**
(43) Date of publication of application: **26.11.2025**
(21) Application number: 24305804.7
(22) Date of filing: 23.05.2024
(51) Int. Cl.: C07K 16/28, A61K 39/395, A61P 35/00

(54) **IL18R AGONIST ANTIBODIES AND USES THEREOF**

(71) Applicant: Egle Therapeutics, 75014 Paris (FR)
(72) Inventor: The designation of the inventor has not yet been filed
(74) Representative: Ipsilon

(57) **Abstract**

The present disclosure relates to isolated agonist anti-IL18R antibodies, or fragments thereof, which selectively inhibits tumor-associated regulatory T cells. The disclosure also relates to isolated nucleic acid comprising sequences encoding the antibodies or fragments thereof. The antibodies and fragments thereof can be used for the prevention and/or treatment of cancer.

## Description

### TECHNICAL FIELD

The present disclosure relates to antibodies, and combinations thereof, targeting IL-18 receptor (IL-18R), and methods using those antibodies.

### BACKGROUND

In an era where cancer remains one of the most pressing challenges to global health, the need for innovative, targeted treatments has never been more acute. The World Health Organization (WHO) estimates that cancer is responsible for nearly 10 million deaths annually worldwide, with incidence rates continuing to rise. Despite significant advances in medical science, traditional cancer treatments such as chemotherapy and radiation therapy often come with debilitating side effects that can severely impact patients' quality of life.

In recent years, the field of oncology has witnessed remarkable progress in our understanding of the molecular mechanisms underlying cancer development and progression. This burgeoning knowledge has revealed a myriad of potential targets for therapeutic intervention, ranging from oncogenes and tumor suppressor genes to signaling pathways and immune checkpoints.

However, despite the wealth of targets identified thus far, many conventional cancer therapies continue to suffer from significant off-target effects, leading to unintended harm to healthy cells and tissues. This not only compromises the efficacy of treatment but also exacerbates the burden of adverse side effects borne by patients.

Indeed, the toxicities associated with traditional cancer treatments pose a formidable barrier to optimal patient care. Chemotherapy, for example, is notorious for its systemic toxicity, resulting in hair loss, nausea, fatigue, and immunosuppression, among other debilitating effects. Similarly, radiation therapy can cause tissue damage in surrounding healthy organs, leading to long-term complications and diminished quality of life.

Against this backdrop, there exists a need to develop targeted therapies that selectively disrupt cancer cell growth and survival while sparing normal tissues from harm. Such therapies hold the promise of achieving superior efficacy with reduced toxicity, thereby revolutionizing the landscape of cancer treatment.

Therefore, there is still a need to identify new molecules highly expressed in the context of cancers while at the same time having a low overall healthy tissue expression.

There is also still a need to provide new treatments, and in particular new antibodies, effective against such specific molecule identified as being differentially expressed in the context of cancer as compared to healthy tissues, and in particular as being significantly more expressed in the context of cancer as compared to healthy tissues.

There is also still a need to provide new treatments effective for preventing and/or treating cancer, in particular for preventing and/or treating cancer with a low, or an absence of, effect on healthy tissues in an individual in need thereof.

There is also a need to provide new treatments effective for preventing and/or treating cancer resistant to current anti-cancer treatments, in particular cancer resistant to the immune checkpoint blockade (ICB) targeting programmed cell death 1/programmed cell death ligand 1 (PD1/PDL1) axis (i.e. anti-PD1/PDL1 therapy).

The present disclosure aims at satisfying all or parts of those needs.

### SUMMARY

According to one of its objects, the present disclosure relates to an isolated agonist anti-II,18R antibody, or a fragment thereof, which selectively inhibits cancer-associated regulatory T cells.

In the last years, interleukin-18 (IL-18) has emerged as a pivotal immune-enhancing cytokine, orchestrating multifaceted effects on various immune cells. It signals through a heterodimeric cell surface receptor composed of the IL-18 receptor (IL-18R) α and β chains (also called IL18R1 and IL18Rap, respectively) (Landy et al., 2024, "Biological and Clinical Roles of IL-18 in Inflammatory Diseases." Nature Reviews Rheumatology 20 (1): 33-47. https://doi.org/10.1038/s41584-023-01053-w), facilitating downstream signaling via adaptor molecules and MYD88 phosphorylation, ultimately activating the MAPK and NF-KB pathways (Ohnishi et al. 2012, "TRAM Is Involved in IL-18 Signaling and Functions as a Sorting Adaptor for MyD88." Edited by Tianyi Wang. PLoS ONE 7 (6): e38423. https://doi.org/10.1371/joumal.pone.0038423). The extracellular activity of IL-18 can be opposed by an abundant, high-affinity 'decoy-receptor' called IL-18 binding protein (IL-18BP), which solely functions to inhibit IL-18 (Novick et al. Immunity. 1999, "Interleukin-18 Binding Protein: A Novel Modulator of the Th1 Cytokine Response." Binding Protein).

Among the described immune functions of IL-18, it can drive the differentiation of naive CD4+ T lymphocytes into Th1 cells, promote the development of memory cytotoxic CD8+ T lymphocytes, and enhance the activation and cytotoxic function of NK cells (Landy et al. 2024).

Only a few studies have investigated the effects of IL-18 directly acting on Treg cells. In the context of obesity, it has been shown that IL-18-treated Tregs have increased proteasomal degradation of FoxP3 and compromised suppressive function (Akimova et al. 2021, "Obesity-Related IL-18 Impairs T-Regulatory Cell Function and Promotes Lung Ischemia-Reperfusion Injury." American Journal of Respiratory and Critical Care Medicine 204 (9): 1060-74. https://doi.org/10.1164/rccm.202012-4306OC). The existing literature however lacks comprehensive exploration of the relationship between IL-18 signaling and regulatory T cells (Tregs) within the context of immuno-oncology.

As shown in the Examples section, the inventors have surprisingly identified IL-18R as being significantly expressed in cancer-infiltrating Treg cells, particularly those exhibiting potent suppressive capabilities, and its suitability as a therapeutic target, considering its minimal expression in healthy tissues.

Moreover, the inventors have developed agonist antibodies against IL-18R having the ability to selectively inhibit said Tregs in cancers. Treg inhibition by the agonist anti-IL-18R antibodies according to the invention will consequently promote immune responses (lymphocytes (B, NK, CD4+ or CD8+ T cells); dendritic cells (DC); macrophages and others) by unleashing immune cells from Treg suppression, and will accordingly stimulate the immune system against cancers.

Furthermore, the inventors have surprisingly demonstrated, through the enclosed examples, a remarkable finding: not only does II,18R exhibit low expression in healthy tissues, but it also shows significantly heightened expression specifically in cancer-infiltrating Treg cells compared to those present elsewhere in the body. This advantageous characteristic underscores the safety of targeting IL18R for the prevention/treatment of cancers and employing its agonists since unlike other potential agents that might affect Tregs systemically, such targeted therapy ensures that the immune system remains undisturbed throughout the body of treated individuals, averting the risk of undue immune system activation outside of cancers.

An isolated agonist anti-IL18R antibody, or a fragment thereof, according to the invention may in particular comprise:
(a) a heavy chain wherein the variable domain comprises:
   - a H-CDR1 having at least 88% identity with sequence set forth as SEQ ID NO: 1,
   - a H-CDR2 having at least 85% identity with sequence set forth as SEQ ID NO: 2, and
   - a H-CDR3 having at least 90% identity with sequence set forth as SEQ ID NO: 3;
   and
(b) a light chain wherein the variable domain comprises:
   - a L-CDR1 having at least 80% identity with sequence set forth as SEQ ID NO: 5,
   - a L-CDR2 having the sequence DTS, and
   - a L-CDR3 having at least 88% identity with sequence set forth as SEQ ID NO: 7.

An antibody or fragment thereof according to the invention may comprise (a) a heavy chain wherein the variable domain comprises SEQ ID NO:1 as H-CDR1, SEQ ID NO:2 as H-CDR2 and SEQ ID NO:3 as H-CDR3; and (b) a light chain wherein the variable domain comprises SEQ ID NO: 5 as L-CDR1, DTS as L-CDR2 and SEQ ID NO:7 as L-CDR3.

An antibody or fragment thereof according to the invention may comprise:
- a heavy chain variable domain having at least 85 %, in particular at least 90%, identity with SEQ ID NO: 4; and
- a light chain variable domain having at least 85 %, in particular at least 90%, identity with SEQ ID NO: 8.

A fragment of antibody according to the invention may be selected from the group consisting of Fv, Fab, F(ab')2, Fab', dsFv, scFv, sc(Fv)2 and diabodies.

An antibody or fragment thereof according to the invention may be a multi-specific antibody, and in particular a bispecific antibody, more particularly a bispecific antibody binding to two different epitopes of IL 18R.

An antibody or fragment thereof according to the invention may be fused to an IL-2 variant which is a regulatory T cell (Treg)-specific IL-2 receptor antagonist, in particular may be fused by the intermediate of a linker.

Another object of the present invention relates to an isolated nucleic acid sequence comprising a sequence encoding a heavy chain and/or a light chain of an antibody, or antibody fragment thereof, according to the invention, and in particular encoding an antibody, or antibody fragment thereof, according to the invention.

A further object of the invention relates to a pharmaceutical composition comprising, in a pharmaceutically acceptable carrier, an antibody, or fragment thereof, according to the invention.

Another object of the invention relates to a pharmaceutical composition according to the invention, or an antibody, or fragment thereof, according to the invention, for use in the prevention and/or treatment of cancer in an individual in need thereof.

The individual may in particular be a human being.

The cancer to be prevented and/or treated may be selected from the group consisting of: Renal Cancer, in particular Renal Cell Carcinoma; Colorectal Cancer; Liver Cancer, in particular Hepatocellular Carcinoma; Lung cancer, in particular Non-Small Cell Lung Cancer (NSCLC); Mesothelioma, in particular Malignant Pleural Mesothelioma; Esophageal Cancer; Head and Neck cancer, in particular Head and Neck Squamous Cell Carcinoma or Cervical Cancer; Urothelial Cancer, in particular Urothelial Carcinoma; Lymphoma, in particular Primary Hodgkin Lymphoma or Large B Cell Lymphoma; Gastric Cancer; Skin cancer, in particular Melanoma, Merkel Cell Carcinoma or Cutaneous Squamous Cell Carcinoma; Endometrial Cancer, in particular Endometrial Carcinoma; Breast Cancer, in particular Triple Negative Breast Cancer or Breast invasive Carcinoma; Pancreas cancer, in particular Pancreatic Adenocarcinoma; Thymoma, Prostate Cancer, in particular Prostate Adenocarcinoma; Ovarian Cancer, in particular Ovarian Serous Cystadenocarcinoma; Thyroid Cancer, in particular Thyroid Carcinoma, Glioblastoma and Sarcoma. It may in particular be selected from the group consisting of Lung cancer, in particular Non-Small Cell Lung Cancer (NSCLC) and Breast Cancer.

The pharmaceutical composition according to the invention, or the antibody, or fragment thereof, according to the invention, may be administered to the individual in need thereof in combination with at least one immune check-point inhibitor; preferably anti-PD-1 and/or anti-PDL-1.

The pharmaceutical composition according to the invention, or the antibody, or fragment thereof, according to the invention, may be administered to the individual in need thereof in combination with at least one agonist of the IL-12 pathway in regulatory T cells and in particular in the cancer-associated regulatory T cells, and is in particular administered to the individual in need thereof in combination with at least one agent selected from the group consisting of IL-12; an IL-12 receptor activator, such as a recombinant IL-12 (see Bhalchandra Mirlekar and Yuliya Pylayeva-Gupta, Cancers (Basel) 2021 Jan 6;13(2):167) or a drug under development such as DF6002, dodekin, IMNN-001, PDS01ADC, SON-1010, XTX-301, ANK-101, CLN-617, WTX-330, XmAb-662, PD1-IL12, ATP-IL12, split-IL-12, InC-01, SON-1210 and ZW-270.

An agonist antibody or fragment thereof disclosed herein binds to the IL18 receptor and inhibits cancer-infiltrating Treg.

### BRIEF DESCRIPTION OF THE FIGURES

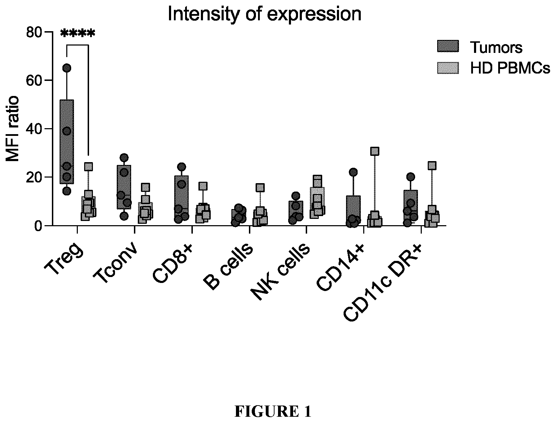

**FIGURE 1** represents the intensity of expression of IL18R in various immune cell types either in the context of healthy PBMC or in the context of cancer. Ordinate: MFI ratio. Abscissa: from left to right: Treg, Tconv, CD8+ cells (CD8+), B cells, NK cells, CD14+ cells (CD14+) and CD11cDR+ cells (CD11cDR+). For each cellular type on the abscissa: the left part shows the results in cancers ("Tumors"), the right part shows the results in healthy donor PBMCs (HD PBMCs). Data representative of 5 independent experiments. Two-way ANOVA with Bonferroni's test for multiple comparisons. P-values ****<0.0001.

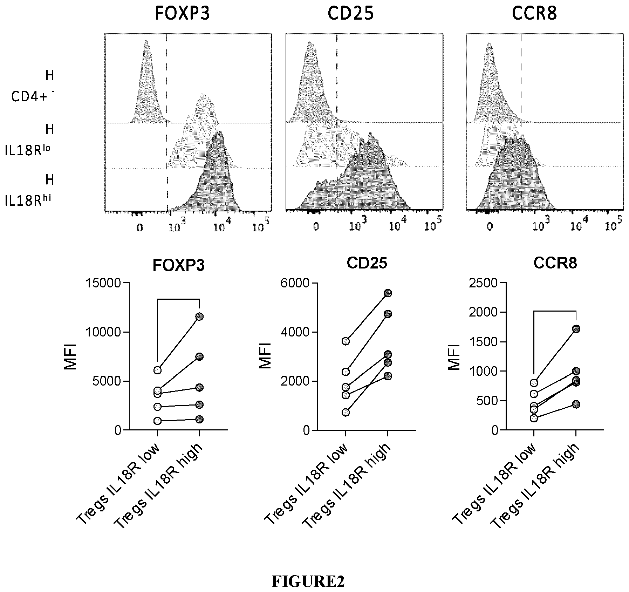

**FIGURE 2** represents the intensity of expression of FOXP3, CD25 and CCR8 in Treg cells expressing low IL18R when compared to Treg cells expressing high IL18R levels. The upper part shows a representative flow-cytometry histogram depicting the levels of expression of the three analyzed molecules in Tconv, Tregs IL18R low and Tregs IL18R high and the lower part represents the quantified expression.

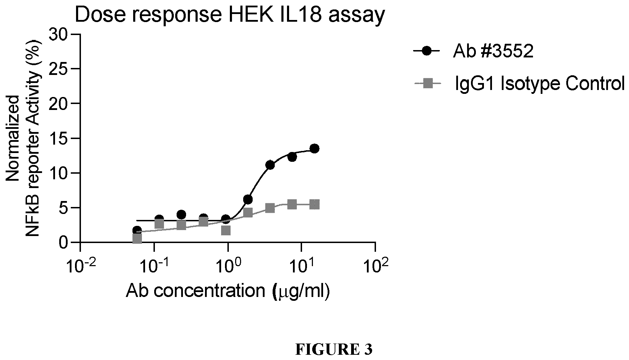

**FIGURE 3** represents the agonist activity of anti-IL18R antibody #3552 according to the invention. The normalized NFkB reporter activity (% - ordinate) of this antibody as compared to the maximun activity observed by the recombinant IL-18 and of a control IgG1 isotype is measured in function of the concentration of the antibodies (nM - abscissa).

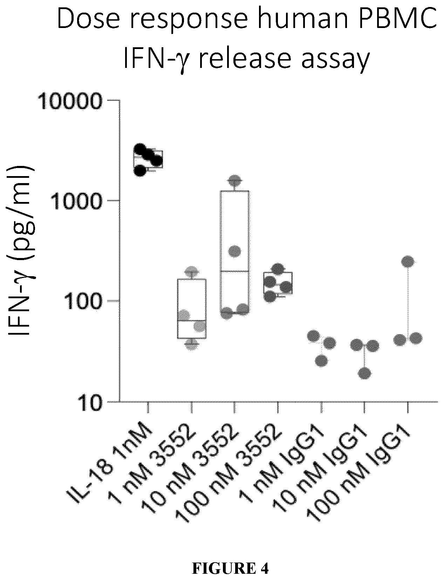

**FIGURE 4** represents agonist activity of anti-IL18R antibody #3552 according to the invention. The production of IFNγ (pg/mL - ordinate) by PBMCs of this antibody and of a control IgG isotype is measured in function of the concentration of the antibodies (nM - abscissa): from the left to the right IL-18 1 nM; 1nM #3552; 10nM #3552; 100nM #3552; 1nM IgG1; 10nM IgG1; 100nM IgG1.

### BRIEF DESCRIPTION OF THE SEQUENCES

SEQ ID NO: 1 represents the amino acids sequence of the HCDR1 of antibody #3552.
SEQ ID NO: 2 represents the amino acids sequence of the HCDR2 of antibody #3552.
SEQ ID NO: 3 represents the amino acids sequence of the HCDR3 of antibody #3552.
SEQ ID NO: 4 represents the amino acids sequence of the heavy variable chain of antibody #3552.
SEQ ID NO: 5 represents the amino acids sequence of the LCDR1 of antibody #3552.
SEQ ID NO: 6 represents the amino acids sequence of the LCDR2 of antibody #3552.
SEQ ID NO: 7 represents the amino acids sequence of the LCDR3 of antibody #3552.
SEQ ID NO: 8 represents the amino acids sequence of the light variable chain of antibody #3552.

### DETAILED DESCRIPTION

The present disclosure is not limited to the particular methodology, protocols, cell lines, vectors, or reagents described herein since they may vary without departing from the spirit and scope of the disclosure. Further, the terminology used herein is for the purpose of exemplifying particular embodiments only and is not intended to limit the scope of the disclosure. Unless defined otherwise, all technical and scientific terms and any acronyms used herein have the same meanings as commonly understood by one of ordinary skill in the art in the field of the disclosure. Any method and material similar or equivalent to those described herein can be used in the practice of the present disclosure and only exemplary methods, devices, and materials are described herein.

All patents and publications mentioned herein are incorporated by reference for the purpose of describing and disclosing the antibodies, vectors, nucleic acids, host cells and methodologies reported therein that might be used with and in the present disclosure.

Prior to teaching the making and using of the antibodies binding a II,18 receptor, or an antigen-binding fragment thereof, and combinations of such antibodies, related methods and products of interest, the following non-limiting definitions of some terms and phrases are provided.

### Definitions

Unless otherwise defined herein, scientific and technical terms used in connection with the present invention shall have the meanings that are commonly understood by those of ordinary skill in the art. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, may provide one of skill with a general dictionary of many of the terms used in this disclosure. In case of conflict, the present specification, including definitions, will control. Exemplary methods and materials are described below, although methods and materials similar or equivalent to those described herein can also be used in the practice or testing of the present invention. Units, prefixes, and symbols are denoted in their International System of Units (SI) accepted form. The headings provided herein are not limitations of the various aspects of the disclosure.

Numeric ranges are inclusive of all the numbers defining the range. Unless otherwise indicated, amino acid sequences are written left to right in amino to carboxy orientation.

All publications and other references mentioned herein are incorporated by reference in their entirety.

It is to be noted that the term "**a**" or "**an**" entity refers to one or more of that entity; for example, "a nucleotide sequence," is understood to represent one or more nucleotide sequences. As such, the terms "**a**" (or "**an**"), "**one or more**" and "**at least one**" can be used interchangeably herein.

Furthermore, "**and/or**" where used herein is to be taken as specific disclosure of each of the two specified features or components with or without the other. Thus, the term "and/or" as used in a phrase such as "A and/or B" herein is intended to include "A and B", "A or B", "A" (alone), and "B" (alone).

It is understood that aspects and embodiments of the present disclosure described herein include "**comprising**", "**having**", "**consisting of**", and "**consisting essentially of**" aspects and embodiments. The words "have" and "comprise," or variations such as "has," "having", "comprises", or "comprising", will be understood to imply the inclusion of the stated element(s) (such as a composition of matter or a method step) but not the exclusion of any other elements. The term "consisting of" implies the inclusion of the stated element(s), to the exclusion of any additional elements. The term "consisting essentially of" implies the inclusion of the stated elements, and possibly other element(s) where the other element(s) do not materially affect the characteristic(s) of the stated elements. It is understood that the different embodiments of the disclosure using the term "comprising" or equivalent cover the embodiments where this term is replaced with "consisting of" or "consisting essentially of".

As used herein the term "**antibody**" have the same meaning and will be used equally in the present description. The term "antibody" as used herein refers to isolated or recombinant immunoglobulin molecules and immunologically active portions of immunoglobulin molecules, i.e., molecules that contain an antigen binding site that immunospecifically binds an antigen, such as IL18R. As such, the term antibody encompasses not only whole antibody molecules, but also antibody fragments as well as variants (including derivatives) of antibodies and antibody fragments. In natural antibodies, two heavy chains are linked to each other by disulphide bonds and each heavy chain is linked to a light chain by a disulphide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Complementarity Determining Regions or CDRs refer to amino acid sequences which together define the binding affinity and specificity of the natural Fv region of a native immunoglobulin binding site. The light and heavy chains of an immunoglobulin each have three CDRs, designated CDR1-L, CDR2-L, L- CDR3-L and CDR1-H CDR2-H, CDR3-H, respectively. An antigen-binding site, therefore, typically includes six CDRs, comprising the CDR set from each of a heavy and a light chain V region. Framework Regions (FRs) refer to amino acid sequences interposed between CDRs.

The term "antibody" includes, but is not limited to, monoclonal antibodies, human antibodies, humanized antibodies, camelid antibodies and chimeric antibodies. The antibodies can be of any isotype/class (e.g., IgG, IgE, IgM, IgD, IgA and IgY), or subclass (e.g., IgGl, IgG2, IgG3, IgG4, IgAl and IgA2). In some embodiments, an antibody of the present disclosure refers to an anti-IL18R antibody, i.e. an antibody binding an epitope of any of α (also called IL18R1) or β (also called IL18Rap) chains from IL18R, more particularly an antibody binding an epitope of any of the extracellular domain of chain α or the extracellular domain of chain β from IL 18R.

In the context of the present disclosure, the term *"antibody"* includes monoclonal anti-IL18R antibodies and multi-specific anti-IL18R antibodies.

It is intended that all references to the term "antibody" or "antigen-binding fragment thereof' as used herein, referred to the antibodies or the antigen-binding fragments thereof as described in the present disclosure.

An "**isolated**" compound or entity, such as an antibody or antigen-binding fragment thereof, a vector or a cell, is one that has been identified and separated and/or recovered from a component of its natural environment. Contaminant components of its natural environment are materials that would interfere with diagnostic or therapeutic uses for the compound or entity, and may include enzymes, hormones, and other proteinaceous or non-proteinaceous solutes. In some embodiments, the compound or entity is partially or substantially purified. In some embodiments, the compound or entity may be purified to greater than about 95% by weight of antibody as determined by the Lowry method, for example, more than 95%, 96%, 97%, 98% or 99% by weight of compound or entity. In particular, an isolated antibody includes the antibody *in situ* within recombinant cells since at least one component of the antibody's natural environment is not present. Preferably, however, an isolated antibody may be prepared by at least one purification step.

The term "**recombinant**" as applied to an antibody, or an antigen-binding fragment thereof, a nucleic acid sequence, an expression vector or a host cell, means that those are the products of various combinations of *in vitro* cloning, restriction, ligation steps, and other genetic engineering procedures.

The terms "**monoclonal antibody**", "**monoclonal Ab**", "**monoclonal antibody composition**", "**mAb**", or the like, as used herein refer to a preparation of antibody molecules of single molecular composition. A monoclonal antibody composition displays a single binding specificity and affinity for a particular epitope on an antigen.

The expression "**complementarity-determining regions**" or "**CDRs**" interchangeably refers to the hypervariable regions of VL and VH. The CDRs are the target protein-binding site of the antibody chains that harbors specificity for such target protein, in particular IL 18R. There are three CDRs in each human VH (CDR1-H, CDR2-H, CDR3-H) or VL (CDR1-L, CDR2-L, CDR3-L), constituting in total about 15-20% of the variable domains. CDRs can be referred to by their region and order. For example, "VHCDR1", "CDR1-H" or "H-CDR1" refer to the first CDR of the heavy chain variable region. The CDRs are structurally complementary to the epitope of the target protein, such as II,18R, and are thus directly responsible for the binding specificity. The remaining stretches of the VL or VH, i.e., framework regions, exhibit less variation in amino acid sequence. The positions of the CDRs and framework regions can be determined using various well-known definitions in the art, e.g., Kabat, Chothia, IMGT, and AbM.

In the context of the present disclosure, the amino acid residues of the antibody of the disclosure are numbered according to the **IMGT numbering system.** The IMGT unique numbering has been defined to compare the variable domains whatever the antigen receptor, the chain type, or the species (Lefranc M.-P., Immunology Today, 18, 509 (1997) ; Lefranc et al. Dev. Comp. Immunol., 27, 55-77 (2003)). In the IMGT unique numbering, the conserved amino acids always have the same position, for instance cysteine 23, tryptophan 41, hydrophobic amino acid 89, cysteine 104, phenylalanine or tryptophan 118. The IMGT unique numbering provides a standardized delimitation of the framework regions (FR1-IMGT: positions 1 to 26, FR2-IMGT: 39 to 55, FR3-IMGT: 66 to 104 and FR4-IMGT: 118 to 128) and of the complementarity determining regions: CDR1-IMGT: 27 to 38, CDR2-IMGT: 56 to 65 and CDR3-IMGT: 105 to 117. If the CDR3-IMGT length is less than 13 amino acids, gaps are created from the top of the loop, in the following order 111, 112, 110, 113, 109, 114, etc. If the CDR3-IMGT length is more than 13 amino acids, additional positions are created between positions 111 and 112 at the top of the CDR3-IMGT loop in the following order 112.1,111.1, 112.2, 111.2, 112.3, 111.3, etc. (http://www.imgt.org/IMGTScientificChart/Nomenclature/ IMGT-FRCDRdefinition.html).

Both the light and heavy chains are divided into regions of structural and functional homology. The terms "**constant**" and "**variable**" are used functionally. In this regard, it will be appreciated that the variable domains of both the light (VL) and heavy (VH) chain portions determine antigen recognition and specificity. Conversely, the constant domains of the light chain (CL) and the heavy chain (CH1, CH2, or CH3) confer important biological properties such as secretion, transplacental mobility, Fc receptor binding, complement binding, and the like. By convention, the numbering of the constant region domains increases as they become more distal from the antigen binding site or amino-terminus of the antibody. The N-terminus is a variable region and at the C-terminus is a constant region; the CH3 and CL domains actually comprise the carboxy-terminal domains of the heavy and light chain, respectively.

The term "**identity**" or "**homology**" may mean the percentage of nucleotide bases or amino acid residues in the candidate sequence that are identical with the residue of a corresponding sequence to which it is compared, after aligning the sequences and introducing gaps, if necessary, to achieve the maximum percent identity for the entire sequence, and not considering any conservative amino acid substitutions as part of the sequence identity. Neither N-terminal or C-terminal extensions nor insertions shall be construed as reducing identity or homology. Methods and computer programs for the alignment are available and well known in the art. Sequence identity may be measured using sequence analysis software. Two examples of algorithms that are suitable for determining percent sequence identity and sequence similarity are the BLAST and BLAST 2.0 algorithms (the National Center for Biotechnology Information). The BLASTN program is used for nucleotide sequences, whereas the BLASTP program is used for amino acid sequences.

The term "**antigen binding site**" or "**antigen binding region**" as used in the present disclosure refers to the part of the antibody which comprises the area which specifically binds to and is complementary to part or all of an antigen. Where an antigen is large, an antibody may only bind to a particular part of the antigen, which part is termed on epitope. An antigen binding region may be provided by one or more antibody variable domains. Preferably, an antigen binding region is made of the association of an antibody light chain variable domain (VL) and an antibody heavy chain variable domain (VH).

The term "**antigen**" as used in the present disclosure refers to a molecule or a portion of a molecule capable of being bound by one or more antibodies. An antigen can have one or more than one epitope. For instance, in the context of the invention, an antigen may be IL18R, i.e. any of the α (also called IL18R1) or β (also called IL18Rap) chains from IL18R.

The term "**affinity**", as used herein, means the strength of the binding of an antibody to an epitope presented on an antigen. The affinity of an antibody is given by the dissociation constant KD, defined as [Ab] × [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Ka is defined by 1/Kd. Methods for determining the affinity of Abs can be found in, for example, Harlow, et al., *Cold Spring Harbor Laboratory Press, Cold Spring Harbor*, N.Y., 1988, Coligan et al., Current Protocols in Immunology, Greene Publishing Assoc. and Wiley Interscience, N.Y., (1992, 1993), or Müller, Methods Enzymol. 1983;92:589-601, which references are entirely incorporated herein by reference. A preferred and standard method well known in the art for determining the affinity of mAbs is the measurement of surface plasmon resonance) by using the Biacore instruments (Laure et al. Curr Protoc Protein Sci. 2006 Sep;Chapter 19:Unit 19.13). For multimeric antigens, such as virus capsids, to which both antigen binding sites of an antibody can bind simultaneously, Ka and KD values determined by such standard methods measure the **functional affinity**, or **avidity** of the interaction. Illustratively, an antibody is considered to bind an antigen when a functional binding affinity (KD), preferably measured by surface plasmon resonance, is of 10⁻⁸ mol/l (M) or less, preferably 10⁻⁹ M to 10⁻¹² M.

The term "**parenteral administration**" designates administration routes other than enteral and topical administration, generally by injection, by intravenous, intramuscular, intraarterial, intrathecal, intracapsular, intraorbital, intracardiac, intradermal, intraperitoneal, transtracheal, subcutaneous, epidermal, intraarticular, subcapsular, subarachnoid, intraspinal, epidural and intrasternal routes, including, without limitation, injections, and perfusions.

The terms "**treat**", "**treatment**" or "**therapy**" in the present invention refers to the administration or consumption of a compound or composition according to the invention with the purpose to cure, heal, alleviate, relieve, remedy, ameliorate or improve a disorder, the symptoms of the condition, the likelihood of the disorder, or to prevent or delay the onset of the symptoms, complications, or otherwise arrest or inhibit further development of the disorder in a statistically significant manner. More particularly, *"treating"* or *"treatment"* includes any approach for obtaining beneficial or desired results in a subject's cancer. Beneficial or desired clinical results can include, but are not limited to, alleviation or amelioration of one or more symptoms or conditions of cancer, diminishment or reduction of the extent of a cancer or of a symptom-associated cancer, stabilizing, *i.e.**,*** not worsening, the state of a cancer or of a symptom-associated cancer, delay or slowing of a cancer, amelioration or palliation of a cancer state, diminishment of the reoccurrence of cancer, and remission, whether partial or total and whether detectable or undetectable. In other words, "**treatment**" as used herein includes any cure, amelioration, or reduction of a cancer or related symptom. A "**reduction**" of a symptom or a disease means decreasing of the severity or frequency of the disease or symptom, or elimination of the disease or symptom.

As used herein, the terms "**prevent**" or "**preventing**" (and grammatical variants thereof) with respect to a disease or disorder relate to prophylactic treatment of a disease, e.g., in a subject suspected to have the disease, or at risk for developing the disease. Prevention may include, but is not limited to, preventing, or delaying onset or progression of the disease and/or maintaining one or more symptoms of the disease at a desired or sub-pathological level. The term *"prevent"* does not require the 100% elimination of the possibility or likelihood of occurrence of the event. Rather, it denotes that the likelihood of the occurrence of the event has been reduced in the presence of a composition or method as described herein. More particular, "prevent" or "preventing" refers to a decrease in the risk of occurrence of a cancer or symptom in a subject. As indicated above, the prevention may be complete, i.e., no detectable symptoms or disease, or partial, such that fewer symptoms or less severity of the disease are observed than would likely occur absent treatment.

As used herein, the terms "**disorder**" and "**disease**" are used interchangeably herein and comprise any undesired physiological change in a subject or a cell.

The term "**cancer**" as used herein is defined as a disease characterized by the rapid and uncontrolled growth of aberrant cells. Cancer cells can spread locally or through the bloodstream and lymphatic system to other parts of the body. A cancer considered in the present invention may for example be selected from the group consisting of Renal Cancer, in particular Renal Cell Carcinoma; Colorectal Cancer; Liver Cancer, in particular Hepatocellular Carcinoma; Lung cancer, in particular Non-Small Cell Lung Cancer (NSCLC); Mesothelioma, in particular Malignant Pleural Mesothelioma; Esophageal Cancer; Head and Neck cancer, in particular Head and Neck Squamous Cell Carcinoma or Cervical Cancer; Urothelial Cancer, in particular Urothelial Carcinoma; Lymphoma, in particular Primary Hodgkin Lymphoma or Large B Cell Lymphoma; Gastric Cancer; Skin cancer, in particular Melanoma, Merkel Cell Carcinoma or Cutaneous Squamous Cell Carcinoma; Endometrial Cancer, in particular Endometrial Carcinoma; Breast Cancer, in particular Triple Negative Breast Cancer or Breast invasive Carcinoma; Pancreas cancer, in particular Pancreatic Adenocarcinoma; Thymoma, Prostate Cancer, in particular Prostate Adenocarcinoma; Ovarian Cancer, in particular Ovarian Serous Cystadenocarcinoma; Thyroid Cancer, in particular Thyroid Carcinoma, Glioblastoma and Sarcoma.

The term "**inhibits**" as used herein, or variants of this term, in the context of inhibition of cancer-associated regulatory T cells, refers to the reduction or suppression of its normal function or activity, in particular its function or activity of down-regulating the immune response. The reduction of function or activity can be partial or total. When the reduction of function or activity is total, the Treg can either still be alive in an inactivated form or can be dead.

As used herein, the terms "**therapeutically effective amount**" and "**prophylactically effective amount**" refer to an amount that provides a therapeutic benefit in the treatment, prevention, or management of pathological processes considered. The specific amount that is therapeutically effective can be readily determined by an ordinary medical practitioner and may vary depending on factors such as the type and stage of pathological processes considered, the subject's medical history and age, and the administration of other therapeutic agents.

As used herein, the term "**individual in need thereof**", "**patient**" or "**subject**" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). The subject may in particular be a human.

The term "**nucleic acid sequence**" is used herein interchangeably with the term "**polynucleotide**" and refers to deoxyribonucleotides or ribonucleotides and polymers thereof in either single- or double-stranded form. The term encompasses isolated or recombinant nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphor amidates, methyl phosphonates, chiral-methyl phosphonates, 2-O-methyl ribonucleotides, peptide-nucleic acids (PNAs).

The terms "**polypeptide**" and "**protein**" are used interchangeably herein to refer to a sequence of amino acid residues. The terms apply to amino acid sequences in which one or more amino acid residue is an artificial chemical mimetic of a corresponding naturally occurring amino acid, as well as to naturally occurring amino acid sequences and non-naturally occurring amino acid sequence. Unless otherwise indicated, a particular polypeptide sequence also implicitly encompasses conservatively modified variants thereof.

### IL18R

The antibodies described herein specifically bind to the IL18 receptor. Further, the antibodies according to the invention, and fragments thereof, are agonists of this receptor.

In the last years, interleukin-18 (IL-18) has emerged as a pivotal immune-enhancing cytokine, orchestrating multifaceted effects on various immune cells. It signals through a heterodimeric cell surface receptor composed of the IL-18 receptor (IL-18R) α and β chains (also called IL18R1 and IL18Rap, respectively) (Landy *et al*., 2024), facilitating downstream signaling via adaptor molecules and MYD88 phosphorylation, ultimately activating the MAPK and NF-KB pathways (Ohnishi *et al.* 2012). The extracellular activity of IL-18 can be opposed by an abundant, high-affinity 'decoy-receptor' called IL-18 binding protein (IL-18BP), which solely functions to inhibit IL-18 (Novick et al. Immunity. 1999).

Among the described immune functions of IL-18, it can drive the differentiation of naive CD4+ T lymphocytes into Th1 cells, promote the development of memory cytotoxic CD8+ T lymphocytes, and enhance the activation and cytotoxic function of NK cells (Landy et al. 2024).

Only a few studies have investigated the effects of IL-18 directly acting on Treg cells. In the context of obesity, it has been shown that IL-18-treated Tregs have increased proteasomal degradation of FoxP3 and compromised suppressive function (Akimova *et al.* 2021). The existing literature however lacks comprehensive exploration of the relationship between IL-18 signaling and regulatory T cells (Tregs) within the context of immuno-oncology.

As mentioned above and demonstrated in the enclosed examples, the inventors have surprisingly identified IL-18R has being significantly expressed in cancer-infiltrating Treg cells, particularly those exhibiting potent suppressive capabilities, and accordingly its suitability as a therapeutic target considering its minimal expression in healthy tissues.

Moreover, the inventors have developed agonist antibodies against IL-18R having the ability to selectively inhibit said Tregs in cancers since Treg inhibition by the agonist anti-IL-18R antibodies according to the invention stimulates the immune system against cancers through promotion of the immune responses (lymphocytes (B, NK, CD4+ or CD8+ T cells); dendritic cells (DC); macrophages and others) by unleashing immune cells from Treg suppression.

The expressions "**IL18R**" and "**IL18 receptor**" are herein used interchangeably.

### Anti-IL18R antibodies

An object of the invention relates to antibodies, or fragment thereof, binding to IL18R. In the description, except otherwise indicated, "**antibody**" and "**antigen-binding fragment**" are used interchangeably.

The antibodies described herein may be isolated antibodies.

The antibodies described herein may be recombinant antibodies.

The antibodies may be monoclonal or multi-specific antibodies.

The antibodies may be human antibodies or human engineered antibodies.

An antibody may be provided as antigen-binding fragment. Antigen-binding fragments may be Fv, Fab, F(ab')2, Fab', dsFv, (dsFv)2, scFv, sc(Fv)2, or diabodies.

The term "**antigen-binding fragment**" refers to a fragment of an intact antibody that retain the ability to specifically binds to a given antigen/ligand. Examples of binding fragments include a Fab fragment, a monovalent fragment consisting of the VL, VH, CL and CH1 domains; a Fab' fragment, a monovalent fragment consisting of the VL, VH, CL, CH1 domains and hinge region; a F(ab')₂ fragment, a bivalent fragment comprising two Fab' fragments linked by a disulphide bridge at the hinge region; an Fd fragment consisting of VH domains of a single arm of an antibody; a single domain antibody (sdAb) fragment (Ward et al., 1989 Nature 341:544-546), which consists of a VH domain or a VL domain; and an isolated complementary determining region (CDR). Furthermore, although the two domains of the Fv fragment, VL and VH, are coded for by separate genes, they can be joined, using recombinant methods, by an artificial peptide linker that enables them to be made as a single protein chain in which the VL and VH regions pair to form monovalent molecules (known as single chain Fv (ScFv); see, e.g., Bird et al., 1989 Science 242:423-426; and Huston et al., 1988 proc. Natl. Acad. Sci. 85:5879-5883). "dsFv" is a VH::VL heterodimer stabilized by a disulfide bond. Divalent and multivalent antibody fragments can form either spontaneously by association of monovalent scFvs or can be generated by coupling monovalent scFvs by a peptide linker, such as divalent sc(Fv)2. Such single chain antibodies include one or more antigen biding portions or fragments of an antibody. These antibody fragments are obtained using conventional techniques known to those skilled in the art, and the fragments are screened for utility in the same manner as are intact antibodies. A unibody is another type of antibody fragment lacking the hinge region of IgG4 antibodies. The deletion of the hinge region results in a molecule that is essentially half the size of traditional IgG4 antibodies and has a univalent binding region rather than the bivalent biding region of IgG4 antibodies. Further details on UniBodies may be obtained by reference to WO 2007/059782, which is incorporated by reference in its entirety. Antigen binding fragments can be incorporated into single domain antibodies, SMIP, maxibodies, minibodies, intrabodies, diabodies, triabodies and tetrabodies (see, e.g., Hollinger and Hudson, 2005, Nature Biotechnology, 23, 9, 1126-1136). The term "diabodies" "tribodies" or "tetrabodies" refers to small antibody fragments with multivalent antigen-binding sites (2, 3 or four), which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Further details on domain antibodies and methods of their production are found in US 6,291,158; 6,582,915; 6,593,081; 6,172,197; and 6,696,245; US 2004/0110941; EP 1433846, 0368684 and 0616640; WO 2005/035572, 2004/101790, 2004/081026, 2004/058821, 2004/003019 and 2003/002609, each of which is herein incorporated by reference in its entirety. Antigen binding fragments can be incorporated into single chain molecules comprising a pair of tandem Fv segments (VH-CH1-VH-CH1) Which, together with complementary light chain polypeptides, form a pair of antigen binding regions (Zapata et al., 1995 Protein Eng. 8(10); 1057-1062 and U.S. Pat. No. 5,641,870).

Fab fragments can be obtained by treating an antibody with a protease, papain. Also, the Fab can be produced by inserting DNA encoding Fab of the antibody into a vector for prokaryotic expression system, or for eukaryotic expression system, and introducing the vector into a procaryote or eucaryote (as appropriate) to express the Fab.

F(ab')₂ can be obtained by treating an antibody with a protease, pepsin. Also, the F(ab')2 can be produced by binding Fab' described below via a thioether bond or a disulphide bond.

Fab' can be obtained by treating F(ab')2 with a reducing agent, dithiothreitol. Also, the Fab' can be produced by inserting DNA encoding Fab' fragment of the antibody into an expression vector for prokaryote, or an expression vector for eukaryote, and introducing the vector into a prokaryote or eukaryote (as appropriate) to perform its expression.

An antigen-binding fragment may be variable heavy chain of a single domain antibody (VHH).

As used herein, the term and expression "**binding**" or "**specifically binding**" are used interchangeably and refer to the ability of an antibody or antigen-binding fragment thereof to detectably bind an epitope present on a specific antigen, such as a IL18R, while having relatively no or little detectable reactivity with different antigen, for example non-IL18R proteins or structures.

The antibody or antigen-binding fragment thereof forms a complex with an antigen that is relatively stable under physiologic conditions. Specific binding can be characterized by an equilibrium dissociation constant (K_{D}) of at least about 1×10⁻⁶ M or less (e.g., a smaller K_{D} denotes a tighter binding).

Methods for determining whether two molecules specifically bind are well known in the art and include, for example, equilibrium dialysis, surface plasmon resonance, and the like. Moreover, multi-specific antibodies that bind to various epitopes of II,18R or that binds to at least one epitope of IL18R and to one or more additional antigen(s) different from IL18R, in particular a bi-specific antibody that binds to two different epitopes, and more particularly to a first epitope present on chain IL18Rap of II,18R and to a second epitope present on chain IL18R1 of IL18R are considered antibodies that "specifically bind" IL18R according to the invention.

The expression "**agonist anti-IL18R**" intends to refer to an antibody which is capable of specifically binding to an IL18 receptor and to trigger IL18R activation, i.e. triggers IL-18 receptor signaling pathway in the tumor-infiltrating Tregs.

An antibody, or fragment thereof, according to the invention may comprise a heavy chain wherein the variable domain comprises:
- a H-CDR1 having at least 88%, 89%, 90%, 91%, 92%, 93%, 94%, 95%, 96%, 97%, 98%, 99% or 100% identity with sequence set forth as SEQ ID NO: 1,
- a H-CDR2 having at least 85%, 86%, 87%, 88%, 88%, 89%, 90%, 91%, 92%, 93%, 94%, 95%, 96%, 97%, 98%, 99% or 100% identity with sequence set forth as SEQ ID NO: 2, and
- a H-CDR3 having at least 90%, 91%, 92%, 93%, 94%, 95%, 96%, 97%, 98%, 99% or 100% identity with sequence set forth as SEQ ID NO: 3.

An antibody, or fragment thereof, according to the invention may comprise a light chain wherein the variable domain comprises:
- a L-CDR1 having at least 80%, 81%, 82%, 83%, 84%, 85%, 86%, 87%, 88%, 88%, 89%, 90%, 91%, 92%, 93%, 94%, 95%, 96%, 97%, 98%, 99% or 100% identity with sequence set forth as SEQ ID NO: 5;
- a L-CDR2 having the sequence DTS (SEQ ID NO: 6); and
- a L-CDR3 having at least 88%, 89%, 90%, 91%, 92%, 93%, 94%, 95%, 96%, 97%, 98%, 99% or 100% identity with sequence set forth as SEQ ID NO: 7.

An antibody, or fragment thereof, according to the invention may comprise:
(a) a heavy chain wherein the variable domain comprises:
   - a H-CDR1 having at least 88%, 89%, 90%, 91%, 92%, 93%, 94%, 95%, 96%, 97%, 98%, 99% or 100% identity with sequence set forth as SEQ ID NO: 1,
   - a H-CDR2 having at least 85%, 86%, 87%, 88%, 88%, 89%, 90%, 91%, 92%, 93%, 94%, 95%, 96%, 97%, 98%, 99% or 100% identity with sequence set forth as SEQ ID NO: 2, and
   - a H-CDR3 having at least 90%, 91%, 92%, 93%, 94%, 95%, 96%, 97%, 98%, 99% or 100% identity with sequence set forth as SEQ ID NO: 3;
   and
(b) a light chain wherein the variable domain comprises:
   - a L-CDR1 having at least 80%, 81%, 82%, 83%, 84%, 85%, 86%, 87%, 88%, 88%, 89%, 90%, 91%, 92%, 93%, 94%, 95%, 96%, 97%, 98%, 99% or 100% identity with sequence set forth as SEQ ID NO: 5;
   - a L-CDR2 having the sequence DTS (SEQ ID NO: 6); and
   - a L-CDR3 having at least 88%, 89%, 90%, 91%, 92%, 93%, 94%, 95%, 96%, 97%, 98%, 99% or 100% identity with sequence set forth as SEQ ID NO: 7.

An antibody, or fragment thereof, according to the invention may comprise:
(a) a heavy chain wherein the variable domain comprises a CDR1-H comprising an amino acid sequence of SEQ ID NO: 1 or a sequence differing from SEQ ID NO: 1 by one conservative amino acid substitution; a CDR2-H comprising an amino acid sequence of SEQ ID NO: 2 or a sequence differing from SEQ ID NO: 2 by one conservative amino acid substitution; a CDR3-H comprising an amino acid sequence of SEQ ID NO: 3 or a sequence differing from SEQ ID NO: 3 by one conservative amino acid substitution; and
(b) a heavy chain wherein the variable domain comprises a CDR1-L comprising an amino acid sequence of SEQ ID NO: 5 or a sequence differing from SEQ ID NO: 5 by one conservative amino acid substitution, a CDR2-L comprising an amino acid sequence DTS, and a CDR3-L comprising an amino acid sequence of SEQ ID NO: 7 or a sequence differing from SEQ ID NO: 7 by one conservative amino acid substitution.

A "**conservative substitution**" refers to the replacement of an amino acid with another amino acid of similar chemical structure, similar chemical properties or similar side-chain volume in an amino acid sequence. A conservative amino acid substitution will not substantially change the functional properties of a protein. The amino acids introduced may have similar polarity, hydrophilicity or hydrophobicity to the amino acids they replace. Conservative amino acid changes are well known in the art. Conservative amino acid changes may also be determined by reference to the Point Accepted Mutation (PAM) or BLOcks SUbstitution Matrix (BLOSUM) family of scoring matrices for conservation of amino acid sequence. Thus, conservative amino acid changes may be members of an equivalence group, being a set of amino acids having mutually positive scores in the similarity representation of the scoring matrix selected for use in an alignment of the reference and mutant polypeptide chains.

For example, a conservative substitution may be a substitution of an amino acid of one class by an amino acid of the same class as presented in **Table 1.** Alternatively, a conservative substitution may be a substitution of an amino acid of one class by an amino acid of another class but with a similar chemical structure, a similar chemical property and/or a similar side-chain volume. Alternatively, in some embodiments, a substitution mutation may be a non-conservative mutation, which replaces the amino acid of one class with an amino acid of non-similar chemical structure, non-similar chemical property and/or non-similar side-chain volume.

**TABLE 1: Classes of amino acids for conservative substitution**

| **Class** | **Amino acids** | **1-letter code** |
|---|---|---|
| Aliphatic | Glycine, Alanine, Valine, Leucine, Isoleucine | G, A, V, L, I |
| Hydroxyl | Serine, Cysteine, Selenocysteine, | S, C, U, |
| or sulfur/selenium-containing | Threonine, Methionine | T, M |
| Cyclic | Proline | P |
| Aromatic | Phenylalanine, Tyrosine, Tryptophan | F, Y, W |
| Basic | Histidine, Lysine, Arginine | H, K, R |
| Acidic and their amides | Aspartate, Glutamate, Asparagine, Glutamine | D, E, N, Q |

A CDR of an antibody, or antigen-binding fragment thereof, may comprise more than one conservative amino acid substitution, for example at least two, or at least three.

In some embodiments, the full-length heavy chain, the full-length light chain, VH, VL and CDRs of an antibody disclosed herein may comprise at least one conservative substitution.

Percent identity between amino acid sequences may be determined with standard alignment algorithms as those described herein.

For sequence comparison of nucleic acid sequences, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters are used. Methods of alignment of sequences for comparison are well known in the art. Optimal alignment of sequences for comparison can be conducted, e.g., by the local homology algorithm of Smith & Waterman, Adv. Appl. Math. 2:482, 1981, by the homology alignment algorithm of Needleman & Wunsch, J. Mol. Biol. 48:443, 1970, by the search for similarity method of Pearson & Lipman, Proc. Nat'l. Acad. Sci. USA 85:2444, 1988, by computerized implementations of these algorithms (GAP, BESTFIT, FASTA, and TFASTA in the Wisconsin Genetics Software Package, Genetics Computer Group, 575 Science Dr., Madison, Wis.), or by manual alignment and visual inspection (see, e.g., Current Protocols in Molecular Biology (Ausubel et al., eds 1995 supplement)).

One example of a useful algorithm is PILEUP. PILEUP uses a simplification of the progressive alignment method of Feng & Doolittle, J. Mol. Evol. 35:351-360, 1987. The method used is similar to the method described by Higgins & Sharp, CABIOS 5:151-153, 1989. Using PILEUP, a reference sequence is compared to other test sequences to determine the percent sequence identity relationship using the following parameters: default gap weight (3.00), default gap length weight (0.10), and weighted end gaps. PILEUP can be obtained from the GCG sequence analysis software package, e.g., version 7.0 (Devereaux et al., Nuc. Acids Res. 12:387-395, 1984).

Another example of algorithms that are suitable for determining percent sequence identity and sequence similarity are the BLAST and the BLAST 2.0 algorithm, which are described in Altschul et al., J. Mol. Biol. 215:403-410, 1990 and Altschul et al., Nucleic Acids Res. 25:3389-3402, 1977. Software for performing BLAST analyses is publicly available through the National Center for Biotechnology Information (ncbi.nlm.nih.gov). The BLASTN program (for nucleotide sequences) uses as defaults a word length (W) of 11, alignments (B) of 50, expectation (E) of 10, M=5, N=-4, and a comparison of both strands. The BLASTP program (for amino acid sequences) uses as defaults a word length (W) of 3, and expectation (E) of 10, and the BLOSUM62 scoring matrix (see Henikoff & Henikoff, Proc. Natl. Acad. Sci. USA 89:10915, 1989).

In some embodiments, an antibody according to the invention, or a fragment thereof, may comprise:
- a heavy chain comprising the amino acid sequence set forth as SEQ ID NO: 4, or a sequence at least 85%, 86%, 87%, 88%, 89%, 90%, 91%, 92%, 93%, 94%, 95%, 96%, 97%, 98%, or 99% identical thereto; and/or
- a light chain comprising an amino acid sequence from SEQ ID NO: 8, or a sequence at least 85%%, 86%, 87%, 88%, 89%, 90%, 91%, 92%, 93%, 94%, 95%, 96%, 97%, 98%, or 99% identical thereto.

In some embodiments, an antibody according to the invention, or a fragment thereof, may comprise:
- a variable domain of heavy chain of sequence set forth as SEQ ID NO: 4, or a sequence at least 85%, 86%, 87%, 88%, 89%, 90%, 91%, 92%, 93%, 94%, 95%, 96%, 97%, 98%, or 99% identical thereto, and a CDR1-H comprising an amino acid sequence having the sequence set forth as SEQ ID NO: 1, or an amino acid sequence differing from said sequence by at least one conservative amino acid substitution; a CDR2-H comprising an amino acid sequence having the sequence set forth as SEQ ID NO: 2, or an amino acid sequence differing from said sequence by at least one conservative amino acid substitution; and a CDR3-H comprising an amino acid sequence having the sequence set forth as SEQ ID NO: 3, or an amino acid sequence differing from said sequence by at least one conservative amino acid substitution; and/or
- a variable domain of light chain of sequence set forth as SEQ ID NO: 8, or a sequence at least 85%%, 86%, 87%, 88%, 89%, 90%, 91%, 92%, 93%, 94%, 95%, 96%, 97%, 98%, or 99% identical thereto, and a CDR1-L comprising an amino acid sequence having the sequence set forth as SEQ ID NO: 5, or an amino acid sequence differing from said sequence by at least one conservative amino acid substitution; a CDR2-L comprising the amino acid sequence DTS (SEQ ID NO: 6); and a CDR3-L comprising an amino acid sequence having the sequence set forth as SEQ ID NO: 7, or an amino acid sequence differing from said sequence by at least one conservative amino acid substitution.

In some embodiments, an antibody according to the invention, or a fragment thereof, may comprise:
- a variable domain of heavy chain of sequence set forth as SEQ ID NO: 4, or a sequence at least 85%, 86%, 87%, 88%, 89%, 90%, 91%, 92%, 93%, 94%, 95%, 96%, 97%, 98%, or 99% identical thereto, and a CDR1-H comprising an amino acid sequence having the sequence set forth as SEQ ID NO: 1; a CDR2-H comprising an amino acid sequence having the sequence set forth as SEQ ID NO: 2; and a CDR3-H comprising an amino acid sequence having the sequence set forth as SEQ ID NO: 3; and/or
- a variable domain of light chain of sequence set forth as SEQ ID NO: 8, or a sequence at least 85%%, 86%, 87%, 88%, 89%, 90%, 91%, 92%, 93%, 94%, 95%, 96%, 97%, 98%, or 99% identical thereto, and a CDR1-L comprising an amino acid sequence having the sequence set forth as SEQ ID NO: 5; a CDR2-L comprising the amino acid sequence DTS (SEQ ID NO: 6); and a CDR3-L comprising an amino acid sequence having the sequence set forth as SEQ ID NO: 7.

In some embodiments, an antibody according to the invention, or a fragment thereof, herein may comprise:
- a heavy chain comprising the amino acid sequence set forth as SEQ ID NO: 4; and/or
- a light chain comprising an amino acid sequence from SEQ ID NO: 8.

The sequences mentioned herein are reminded in the following **Table 2.**

| **SEQ ID NO:** | **Designation** | **Sequence** |
|---|---|---|
| **1** | H-CDR1 | GYSITSGYY |
| **2** | H-CDR2 | ITYDGIN |
| **3** | H-CDR4 | CATPYYGTSGFAY |
| **4** | Heavy variable domain | DVQLQESGPGLVKFFQSLSLTCSVTGYSITSGYYWNWVRQFPGNKLEWLGY ITYDGINDYNPSLKNRISITRDTSKNQFFLRLNSVTTEDTATYYCATPYYG TSGFAYWGQGTLVTVSA |
| **5** | L-CDR1 | SSVGY |
| **6** | L-CDR2 | DTS |
| **7** | L-CDR3 | QQWSSIPLT |
| **8** | Light variable domain | QIVLTQSPAIMSASPGEKVTMTCSASSSVGYMHWYQQRSGTSPKRWIYDTS KLPSGVPARFSASGSGTSYSLTISSMESEDAATYYCQQWSSIPLTFGAGTK LELK |

In some embodiments, an antibody according to the invention may be the antibody referred to as antibody #3552 in the Examples section.

Antibody #3552 comprises (i) a heavy chain comprising the amino acid sequence set forth as SEQ ID NO: 4 and (ii) a light chain comprising an amino acid sequence from SEQ ID NO: 8.

It is further described an isolated nucleic acid sequence comprising a sequence encoding a heavy chain and/or a light chain of an antibody, or fragment thereof, according to the invention.

Also described is an isolated nucleic acid sequence comprising a sequence encoding an antibody, or fragment thereof, according to the invention.

### Multi-specific antibodies

As previously mentioned, an antibody according to the invention may be in the form of a monoclonal antibody or of a multi-specific antibody.

"**Multi-specific antibodies**" refers to antibodies, or fragments thereof, that have binding specificities for at least two different epitopes of one antigen or for at least two distinct antigens. A multi-specific or a bispecific antibody may comprise at least a first antibody, or an antigen-binding fragment thereof, and at least a second antibody, or an antigen-binding fragment thereof, joined to each other.

In the context of the present invention, a multi-specific antibody, or fragment thereof, according to the invention is able to bind to at least one epitope of II,18R, in particular to at least one extracellular epitope of II,18R, more particularly to at least one extracellular epitope of any one of chains IL18Rap or IL18R1 of IL18R.

In particular, a multi-specific antibody, or fragment thereof, according to the invention is able to bind to at least two different epitopes of II,18R, in particular to at least two extracellular epitopes of II,18R, more particularly to at least two extracellular epitopes of chain IL18Rap and/or of chain IL18R1 of II,18R, even more particularly to at least: one extracellular epitope of chain IL18Rap of IL18R and to one extracellular epitope of chain IL18R1 of IL18R.

A multi-specific antibody, or fragment thereof, according to the invention may be a bispecific antibody.

The expression "**bispecific antibodies** (BsAbs)" refers to molecules which combine the antigen-binding sites of two antibodies within a single molecule. Thus, a bispecific antibody is able to bind two different antigens or to two distinct epitopes of a same antigen, simultaneously. The distinct epitopes may be overlapping epitopes or non-overlapping epitopes.

In particular, a bispecific antibody, or fragment thereof, according to the invention is able to bind to two different epitopes of IL18R, in particular to two extracellular epitopes of IL18R, more particularly to two extracellular epitopes of chain IL18Rap and/or of chain IL18R1 of IL 18R, even more particularly to one extracellular epitope of chain IL 18Rap of IL 18R and to one extracellular epitope of chain IL18R1 of II,18R.

Multi-specific or bispecific antibodies can be prepared as full-length antibodies or antibody fragments.

The multi-specific or bispecific antibody may be an isolated or a recombinant bispecific antibody.

An antibody of the multi-specific or a bispecific antibody can be linked to or coexpressed with another antibody by any known techniques in the art. For example, an antibody or fragment thereof can be functionally linked, e.g., by chemical coupling, genetic fusion, noncovalent association or otherwise, to one or more other antibody or antibody fragment to produce a bispecific or a multi-specific antibody. Specific exemplary bispecific formats that can be used include, without limitation, e.g., scFv-based or diabody bispecific formats, IgG-scFv fusions, dual variable domain (DVD)-Ig, Quadroma, knobs-into-holes, common light chain (e.g., common light chain with knobs-into-holes, etc.), CrossMab, CrossFab, (SEED)body, leucine zipper, Duobody, IgG1/IgG2, dual acting Fab (DAF)-IgG, and Mab2 bispecific formats.

### Immunoconjugates

An antibody, or fragment thereof, according to the invention may be in the form of an immunoconjugate.

An immunoconjugate comprising an antibody, or a fragment thereof, as disclosed herein may be prepared according to any known methods in the art.

An antibody, or a fragment thereof, may be conjugated or linked to detectable label or reporter molecule. Such immunoconjugate may be used as a diagnostic tool for detecting and/or measuring a cancer in a subject in need thereof.

A detectable label or reporter molecule can be a radioisotope, such as ³H, ¹⁴C, ³²P, ³⁵S, or ¹²⁵I; a fluorescent or chemiluminescent moiety such as fluorescein isothiocyanate, or rhodamine; or an enzyme such as alkaline phosphatase, β-galactosidase, horseradish peroxidase, or luciferase.

As used herein, the term "labeled", with regard to the antibody according to the disclosure, is intended to encompass direct labeling of the antibody by coupling (i.e., physically linking) a detectable substance, such as a radioactive agent or a fluorophore (e.g. fluorescein isothiocyanate (FITC) or phycoerythrin (PE) or Indocyanine (Cy5)) to the polypeptide, as well as indirect labeling of the polypeptide by reactivity with a detectable substance.

An antibody may be labelled with a radioactive molecule by any method known to the art. For example, radioactive molecules include but are not limited radioactive atom for scintigraphic studies such as ³H, ¹⁴C, ³²P, ³⁵S, I¹²³, I¹²⁴, or ¹²⁵I, In¹¹¹, Re¹⁸⁶, Re¹⁸⁸, Tc⁹⁹. Polypeptides of the invention may be also labelled with a spin label for nuclear magnetic resonance (NMR) imaging (also known as magnetic resonance imaging, MRI), such as iodine-123, indium-111, fluorine-19, carbon-13, nitrogen-15, oxygen-17, gadolinium, manganese or iron.

The immunoconjugates according to the present invention can be prepared as described in the application WO2004/091668.

The antibodies may be covalently attached, directly or via a cleavable or non-cleavable linker, to at least one detectable probe.

"Linker", as used herein, means a chemical moiety comprising a covalent bond or a chain of atoms that covalently attaches two entities, such as for example a polypeptide to a detectable probe; an antibody, or a fragment thereof, to another antibody, or fragment thereof; an antibody, or fragment thereof, to an effector molecule. Suitable linkers are well known to those of skill in the art and include, but are not limited to, straight or branched-chain carbon linkers, heterocyclic carbon linkers, peptide linkers or repeated polypeptide sequences (such as for example GS repeated several times, for examples from 2 to 10 times, in particular 4 times).

The conjugates may be prepared by *in vitro* methods. In order to link a detectable probe to the antibody, a linking group is used. Suitable linking groups are well known in the art and include disulfide groups, thioether groups, acid labile groups, photolabile groups, peptidase labile groups and esterase labile groups. Conjugation of an antibody of the invention with a detectable probe may be made using a variety of bifunctional protein coupling agents including but not limited to N-succinimidyl pyridyldithiobutyrate (SPDB), butanoic acid 4-[(5-nitro-2-pyridinyl)dithio]-2,5-dioxo-1-pyrrolidinyl ester (nitro-SPDB), 4-(Pyridin-2-yldisulfanyl)-2-sulfo-butyric acid (sulfo-SPDB), N-succinimidyl (2-pyridyldithio) propionate (SPDP), succinimidyl (N-maleimidomethyl) cyclohexane-1-carboxylate (SMCC), iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl)-hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al (1987). Carbon labeled 1-isothiocyanatobenzyl methyldiethylene triaminepentaacetic acid (MX-DTPA) is an exemplary chelating agent for conjugation of radionucleotide to the antibody (WO 94/11026).

The linker may be a "cleavable linker" or a "non-cleavable linker" (for example SMCC linker).

Alternatively, a fusion protein comprising an antibody disclosed herein and a detectable probe may be made, by recombinant techniques or peptide synthesis. The length of DNA may comprise respective regions encoding the two portions of the conjugate either adjacent one another or separated by a region encoding a linker peptide which does not destroy the desired properties of the conjugate.

In general, a conjugate can be obtained by a process comprising the steps of:
(i) bringing into contact an optionally-buffered aqueous solution of an antibody as disclosed herein with solutions of a linker and a detectable probe;
(ii) then optionally separating the conjugate which was formed in (i) from the unreacted antibody.

The aqueous solution of cell-binding agent can be buffered with buffers such as, e.g. potassium phosphate, acetate, citrate or N-2-Hydroxyethylpiperazine-N'-2-ethanesulfonic acid (Hepes buffer).

The reaction temperature is usually comprised between 20 and 40° C. The reaction time can vary from 1 to 24 hours. The reaction between the antibody and the detectable probe can be monitored by size exclusion chromatography (SEC) with a refractometric and/or UV detector. If the conjugate yield is too low, the reaction time can be extended.

A number of different chromatography methods can be used by the person skilled in the art in order to perform the separation of step (ii): the conjugate can be purified e.g. by SEC, adsorption chromatography (such as ion exchange chromatography, IEC), hydrophobic interaction chromatograhy (HIC), affinity chromatography, mixed-support chromatography such as hydroxyapatite chromatography, or high performance liquid chromatography (HPLC). Purification by dialysis or diafiltration can also be used.

As used herein, the term "aggregates" means the associations which can be formed between two or more antibodies, being modified or not by conjugation. The aggregates can be formed under the influence of a great number of parameters, such as a high concentration of cell-binding agent in the solution, the pH of the solution, high shearing forces, the number of bonded dimers and their hydrophobic character, the temperature (see Wang & Gosh, 2008, J. Membrane Sci., 318: 311-316, and references cited therein); note that the relative influence of some of these parameters is not clearly established. In the case of proteins and antibodies, the person skilled in the art will refer to Cromwell et al. (2006, AAPS Journal, 8(3): E572-E579). The content in aggregates can be determined with techniques well known to the skilled person, such as SEC (see Walter et al., 1993, Anal. Biochem., 212(2): 469-480).

After step (i) or (ii), the conjugate-containing solution can be submitted to an additional step (iii) of chromatography, ultrafiltration and/or diafiltration.

The conjugate is recovered at the end of these steps in an aqueous solution.

### Production of antibodies

An antibody disclosed herein may be produced by any technique known in the art, such as, without limitation, any chemical, biological, genetic, or enzymatic technique, either alone or in combination.

An antibody can be synthesized by recombinant DNA techniques well-known in the art. For example, an antibody can be obtained as DNA expression products after incorporation of DNA sequences encoding the antibody into expression vectors and introduction of such vectors into suitable eukaryotic or prokaryotic host cells that will express the desired antibody, from which it can be later isolated using well-known techniques, such as described in the Examples.

A nucleic acid sequence may be used to produce an antibody, or an antigen-binding fragment thereof as described in the disclosure. The nucleic acid sequence may be a DNA or a RNA molecule, which may be included in any suitable expression vector, such as a plasmid, cosmid, episome, artificial chromosome, phage, or a viral vector.

A nucleic acid sequence encoding an antibody, or an antigen-binding fragment thereof, as described may be an isolated nucleic acid sequence or a recombinant nucleic acid sequence.

More particularly, a nucleic acid as described herein may encode the heavy chain (VH), the light chain (VL), the full-length heavy chain, and the full-length light chain of an antibody or fragment thereof according to the present invention. Alternatively, a first nucleic acid sequence as described herein may encode the heavy chain variable domain, in particular the full-length heavy chain, and a second nucleic sequence as described herein may encode the light chain variable domain, in particular the full-length light chain, of an antibody, or fragment thereof, according to the present invention.

A nucleic acid sequence can be produced by de novo solid-phase DNA synthesis or by PCR mutagenesis of an existing sequence encoding an antibody as described herein or its antigen-binding fragment thereof. Direct chemical synthesis of nucleic acids can be accomplished by methods known in the art, such as the phosphotriester method of Narang et al, Meth. Enzymol. 68:90, 1979; the phosphodiester method of Brown / al, Meth. Enzymol. 68: 109, 1979; the diethylphosphoramidite method of Beaucage et al, Tetra. Lett., 22: 1859, 1981; and the solid support method of U.S. Patent No. 4,458,066.

Introducing mutations to a polynucleotide sequence by PCR can be performed as described in, *e.g*., PCR Technology: Principles and Applications for DNA Amplification, H.A. Erlich (Ed.), Freeman Press, NY, NY, 1992; PCR Protocols: A Guide to Methods and Applications, Innis et al. (Ed.), Academic Press, San Diego, CA, 1990; Mattila et al, Nucleic Acids Res. 19:967, 1991; and Eckert et al, PCR Methods and Applications 1: 17, 1991.

The invention also relates to expression vectors and host cells for producing the anti-IL18R antibodies according to the invention.

An expression vector may be an isolated or a recombinant expression vector.

A host cell may be an isolated or a recombinant host cell.

An expression vector may comprise a nucleic acid sequence as described herein. Such a vector may comprise regulatory elements, such as a promotor, enhancer, terminator, and the like, to cause or direct expression of said antibody upon transfection into the host cell.

The choice of vector will depend upon a variety of factors such as the type of host cell in which propagation is desired and the purpose of propagation. Some vectors are useful for amplifying and making large amounts of the desired DNA sequence. Other vectors are suitable for expression in cells in culture. Still other vectors are suitable for transfer and expression in cells in a whole animal. The choice of appropriate vector is well within the skill of the art. Many such vectors are available commercially.

Any expression vector for animal cell can be used, so long as a gene encoding the human antibody C region can be inserted and expressed. Examples of suitable vectors include pAGE107, pAGE103, pHSG274, pKCR, pSG1, pCAG and the like. Other examples of vectors include replicating plasmids comprising an origin of replication, or integrative plasmids, such as for instance pUC, pcDNA, pBR, and the like. Other examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, Gpenv+ cells, 293 cells, RS cells, etc. A detailed protocol for producing such replication-defective recombinant viruses may be found for instance in Pastrana et al. (PloS Pathog. 2012, 8, e1002650).

Examples of promoters and enhancers used in the expression vector for animal cell include early promoter and enhancer of SV40, LTR promoter and enhancer of Moloney mouse leukemia virus, promoter and enhancer of immunoglobulin H chain and the like.

Vectors can be prepared by, for example, inserting a polynucleotide of interest into a construct backbone, typically by means of DNA ligase attachment to a cleaved restriction enzyme site in the vector. Alternatively, the desired nucleotide sequence can be inserted by homologous recombination or site-specific recombination. Typically, homologous recombination is accomplished by attaching regions of homology to the vector on the flanks of the desired nucleotide sequence, while site-specific recombination can be accomplished through use of sequences that facilitate site-specific recombination (e.g., cre-lox, att sites, etc.). Nucleic acid containing such sequences can be added by, for example, ligation of oligonucleotides, or by polymerase chain reaction using primers comprising both the region of homology and a portion of the desired nucleotide sequence.

Expression vectors generally have convenient restriction sites located near the promoter sequence to provide for the insertion of nucleic acid sequences encoding proteins of interest. A selectable marker operative in the expression host may be present to facilitate selection of cells containing the vector. In addition, the expression construct may include additional elements. For example, the expression vector may have one or two replication systems, thus allowing it to be maintained in organisms, for example in mammalian or insect cells for expression and in a prokaryotic host for cloning and amplification. In addition, the expression construct may contain a selectable marker gene to allow the selection of transformed host cells. Selection genes are well known in the art and will vary with the host cell used.

In some embodiments, vectors encoding both the heavy and light chains may be coexpressed in the host cell for expression of the entire antibody.

The expression vector is transferred to a host cell by conventional techniques and the transfected cells then are cultured by conventional techniques to produce an antibody or an antigen-binding fragment thereof of the present disclosure.

Isolated or recombinant host cells may be transfected, infected, or transformed by a nucleic acid sequence and/or an expression vector as disclosed herein.

Such host cells may be used for the production of an antibody as described herein.

The term "**transformation**" means the introduction of a "foreign" (i.e., extrinsic, or extracellular) gene, DNA or RNA sequence to a host cell, so that the host cell will express the introduced gene or sequence to produce a desired substance, typically a protein or enzyme coded by the introduced gene or sequence. A host cell that receives and expresses introduced DNA or RNA has been "transformed".

Thus, the present disclosure further provides a recombinant, or isolated, host cell transformed with a nucleic acid sequence encoding an antibody or antigen-binding fragment thereof as disclosed herein, or with an expression vector comprising such nucleic acid sequence.

Any of a number of suitable host cells can be used in the production of an antibody or antigen-binding fragment thereof. In general, the antibody or antigen-binding fragment thereof described herein may be expressed in prokaryotes or eukaryotes (eukaryotic unicellular organism), e.g., bacteria such as *Escherichia coli*, yeasts, such as *Saccharomyces sp.*, or mammalian cell lines, such as Vero cells, HEK293, or CHO, in accordance with conventional techniques.

Suitable bacteria include but are not limited to BL21 Competent E. coli, BL21(DE3) Competent E. coli, NEB Express Competent E. coli, NEB Express Iq Competent E. coli, T7 Express Competent E. coli, T7 Express Iq Competent E. coli, T7 Express lysY Competent E. coli, T7 Express lysY/Iq Competent E. coli, T7 Express Crystal Competent E. coli, SHuffle Express Competent E. coli, SHuffle T7 Express Competent E. coli, SHuffle T7 Express lysY Competent E. coli, SHuffle T7 Competent E. coli, NiCo21(DE3) Competent E. coli, Lemo21(DE3) Competent E. coli.

Suitable mammalian cell lines include, but are not limited to, HeLa cells (e.g., American Type Culture Collection (ATCC) No. CCL-2), CHO cells (e.g., ATCC Nos. CRL9618, CCL61, CRL9096), 293 cells (e.g., ATCC No. CRL-1573), Vero cells, NIH 3T3 cells (e.g., ATCC No. CRL-1658), Huh-7 cells, BHK cells (e.g., ATCC No. CCL10), PC12 cells (ATCC No. CRL1721), COS cells, COS-7 cells (ATCC No. CRL1651), RATI cells, mouse L cells (ATCC No. CCLI.3), human embryonic kidney (HEK) cells (ATCC No. CRL1573), HLHepG2 cells, and the like.).

The antibody or antigen-binding fragment thereof can be prepared in substantially pure or substantially isolated form. Purified antibody or antigen-binding fragment thereof can be provided such that the antibody or antigen-binding fragment thereof is present in a composition that is substantially free of other contaminant components such as enzymes, hormones, and other proteinaceous or non-proteinaceous solutes.

### Pharmaceutical compositions

An antibody, or an antigen-binding fragment thereof, according to the invention may be formulated in pharmaceutical composition with a pharmaceutically acceptable carrier.

A pharmaceutical composition disclosed herein may be used for treating and/or preventing a cancer.

A pharmaceutical composition accordingly comprises an antibody, or an antigen-binding fragment thereof, according to the invention and a pharmaceutically acceptable carrier or excipient.

A pharmaceutical composition may be prepared according to a method comprising at least a step of combining an antibody, or an antigen-binding fragment thereof, according to the invention with a pharmaceutically acceptable carrier or excipient.

Combination of antibodies, or fragments thereof, according to the invention may be formulated to be simultaneously, separately, or sequentially administered in a subject in need thereof, either in the same pharmaceutical composition or in different pharmaceutical compositions according to the invention.

Simultaneous administration intends to refer to an administration of the antibodies or fragments thereof at the same time and at the same location of the subject's body.

Separate administration intends to refer to an administration of the antibodies at the same time and at separate locations of the body, e.g., in the arm and in the limb, or by separate routes, e.g., intramuscular and intravenous. In a separate administration, the administration of different antibodies may be separated by a few seconds or a few minutes.

Sequential administration intends to refer to the administration of the antibodies at different times, either at the same location or at different locations, or via the same or different routes. In a sequential administration, the different antibodies may be administered with a period of time between each administration ranging, for example, from about 30 min, or 1, 2 or 3 hours to about 24 hours, or 1, 2 or 3 days.

As used herein, the expression *"pharmaceutically acceptable carrier or excipient"* designates any substance or composition compatible with the organism of the subject to whom an antibody of the disclosure is to be administered. A pharmaceutically acceptable carrier is a substance or composition the administration of which to a subject does not come with significant deleterious effects such as adverse, allergic or other untoward reaction when administered to a subject. It can be mentioned, for example, non-toxic solvents, dispersion media, coatings, lubricating agents, saccharides, sugar alcohols, polymers, antibacterial and antifungal agents, and the like that are physiologically compatible. Examples of suitable carriers include one or more of a sterile water, a saline aqueous solution, sucrose, saccharose, starches, sorbitol, PVP or PEG, magnesium stearate, preservatives, dyeing agents or flavors. Particularly, such a carrier or vehicle is compatible with oral, parenteral, or rectal administration, and is preferably adapted to parenteral administration.

The form of the pharmaceutical compositions, the route of administration, the dosage and the regimen naturally depend upon the condition to be treated, the severity of the diseases, the age, weight, gender of the subject, etc.

The pharmaceutical compositions of the invention can be formulated for a topical, oral, parenteral, intranasal, intravenous, intramuscular, subcutaneous or intraocular administration and the like.

The pharmaceutical composition can be formulated in the form of a solution, microemulsion, solution, microemulsion, liposome or other ordered structure suited for high medicament concentration.

The pharmaceutical compositions described herein can also include a pharmaceutically acceptable antioxidant. Examples of pharmaceutically acceptable antioxidants comprise (1) water-soluble antioxidants such as ascorbic acid, cysteine hydrochloride, sodium bisulfate, sodium metabisulfite, sodium sulfite, etc., (2) oil-soluble antioxidants, oxidizing agents such as ascorbyl palmitate, butylated hydroxyanisole (BHA), butylated hydroxytoluene (BHT), lecithin, propyl gallate, alpha-tocopherol and others; and (3) metal chelators such as citric acid, ethylenediaminetetraacetic acid (EDTA), sorbitol, tartaric acid, phosphoric acid and others are included.

Examples of suitable aqueous or non-aqueous carriers that can be used in the pharmaceutical compositions described herein comprise water, ethanol, polyols (for example, glycerol, propylene glycol, polyethylene glycol, etc.) and suitable mixtures thereof, vegetable oils such as olive oil and injectable organic esters such as ethyl oleate. Suitable flowability can be maintained, for example, conserving the required particle size in the case of a dispersion and using surfactants. These compositions can also contain adjuvants such as preservatives, wetting agents, emulsifying agents, and dispersing agents. The prevention of the presence of microorganisms can be ensured by both sterilization methods and the inclusion of several antibacterial and antifungal agents such as parabens, chlorobutanol, phenol, sorbic acid and others. It can also be desirable to include isotonic agents, such as sugars, sodium chloride, in the composition. Furthermore, the inclusion of absorption-retardant agents, such as aluminum monostearate and gelatin, can delay the absorption of injectable pharmaceutical forms.

Pharmaceutically acceptable carriers comprise sterile aqueous solutions or dispersions and sterile powders for the extemporary preparation of sterile injectable solutions or dispersions. The use of such carriers and agents for pharmaceutically active substances is well known in the field. Except where a conventional medium or agent is not compatible with the antibody, the use thereof in the pharmaceutical compositions described herein is contemplated. Complementary active substances can also be incorporated in the compositions.

The sterile injectable solutions can be prepared including the antibody, in a required amount in a suitable solvent, potentially with one or a combination of ingredients listed hereinafter, then sterilizing by means of microfiltration. Generally, the dispersions are prepared incorporating the active compound in a sterile vehicle containing a basic dispersion medium and any other necessary ingredients from those listed hereinafter. In the case of sterile powders for the preparation of sterile injectable solutions, the preferred preparation method is a method of drying under vacuum, wherein an antibody powder plus any other desired ingredient is prepared from the previously sterilized and filtered solution, and lyophilizing.

The amount of antibody which can be combined with carrier materials to prepare a unit dosage form can vary according to the subject to be treated and the particular mode of administration. The amount of antibody which can be combined with a carrier material to prepare a unit dosage form, will generally be the amount of the composition which causes a therapeutic effect.

According to some embodiments, the amount of antibody may be about 0.01% to about 99% with respect to the final pharmaceutical composition amount, preferably about 0.1% to about 70% with respect to the final pharmaceutical composition amount, in combination with a pharmaceutically acceptable carrier.

Dosage schedules are adjusted in order to obtain the desired optimal response (for example, a therapeutic response). According to some embodiments, a single bolus administration is possible, and several divided doses can be administered over a long period of time, or the dose can be proportionally reduced or increased as indicated in a situation of imminent treatment. Preferably, the formulation of parenteral compositions is in the form of unit doses, specifically in order to facilitate administration and dosage uniformity.

According to some embodiments, a unit dosage form designates a physical unit which is convenient as a unit dose for the subject to be treated; each unit, associated with the required pharmaceutical carrier, causes the desired therapeutic effect.

According to some embodiments, the antibody may be formulated within a pharmaceutical composition to be administered at the dosage ranges between about 0.0001 mg and 100 mg, more generally between 0.01 mg and 5 mg per kg of body weight of the subject. For instance, the dosage is comprised between 0.3 mg/kg of body weight, 1 mg/kg of body weight, 3 mg/kg of body weight, 5 mg/kg of body weight or 10 mg/kg of body weight, or 1-10 mg/kg.

The selected dosage level depends on the particular composition being used, the route of administration, the time of administration, the elimination rate of the particular compound being used, the duration of treatment, other medicaments, compounds and/or substances used in combination with a particular composition, the age, sex, weight, condition, general health and medical history of the subject to be treated, as well as similar factors well known in the medical field. The dosage level can also vary depending on several pharmacokinetic factors.

The pharmaceutical composition may be administered by means of medical devices known in the field. According to some particular embodiments, the therapeutic compositions according to the disclosure include the devices: needleless hypodermic injection as described in US 5,399,163, pre-filled ampule and non-invasive hypodermic injection as described in US 4,941,880; a non-invasive hypodermic injection as described in US 4,790,824; or a hypodermic injection device without a needle such as the device described in US 4,596,556.

The pharmaceutical compositions described herein can also be administered in combination therapy, that is, in combination with other agents. For example, the combination therapy can include an antibody, or antigen-binding fragments thereof, or a combination of antibody described herein in combination with at least another antiviral agent.

### Therapeutic methods and uses

An antibody, or an antigen-binding fragment thereof, or a pharmaceutical composition according to the invention may be for use as a medicament, in particular for use in the prevention and/or treatment of a cancer in an individual in need thereof.

An antibody, or an antigen-binding fragment thereof, or a pharmaceutical composition according to the invention may be administered to the individual in need thereof in combination with at least one immune check-point inhibitor, preferably an anti-PD-1 and/or anti-PDL-1, in particular an antibody anti-PD-1 and/or an antibody anti-PDL-1.

Examples of an antibody capable of modulating the immune checkpoint protein PD-1 (anti-PD-1) may be human or humanized antibodies selected from the group consisting of pembrolizumab, nivolumab, cemiplimab, IB 1308, BCD-100, PDR001, tislelizumab, camrelizumab, pidilizumab and lambrolizumab (e.g. disclosed as hPD109A and its humanized derivatives h409All, h409A16 and h409A17 in WO2008/156712) and soluble PD-1 ligands including without limitation PD-L2 Fc fusion protein (also known as B7-DC-Ig or AMP-244).

Examples of said antibody capable of modulating the immune checkpoint protein PD-L1 (anti-PD-Ll) may be antibodies selected from the group consisting of durvalumab, avelumab, atezolizumab, MEDI-4736, MPDL328 OA and MIH1 (Affymetrix).

An antibody, or an antigen-binding fragment thereof, or a pharmaceutical composition according to the invention may be administered to the individual in need thereof in combination with at least one agonist of the IL-12 pathway in regulatory T cells, and in particular in the tumor-associated regulatory T cells, and may in particular be administered to the individual in need thereof in combination with at least one agent selected from the group consisting of IL-12; an IL-12 receptor activator, such as a recombinant IL-12 or a drug under development such as DF6002, dodekin, IMNN-001, PDS01ADC, SON-1010, XTX-301, ANK-101, CLN-617, WTX-330, XmAb-662, PD1-IL12, ATP-IL12, split-IL-12, InC-01, SON-1210 and ZW-270.

DF6002 (also known as BMS-986415) is a monovalent IL-12 immunoglobulin Fc fusion protein (see for example Eva Gutierrez et al. Med. 2023 May 12;4(5):326-340.e5). Dodekin (also known as IL12-L19L19) is an immunocytokines, and in particular a vascular targeting antibody fused to IL-12 (see for example Yong Fu et al. Front Immunol. 2023; 14: 1218082).

IMNN-001 (formerly known as Gen-1) is an IL-12 gene-mediated immunotherapy under clinical development by Imunon and currently in Phase II for Epithelial Ovarian Cancer (see for example Amir A. Jazaeri et al. Med. 2023 Nov 10;4(11):755-760).

PDS01ADC (formerly PDS0301, M9241 and NHSIL12) is a novel investigational Interleukin 12 (IL-12) fused antibody-drug conjugate (ADC) that enhances the proliferation, potency and longevity of T cells in the tumor microenvironment.

SON-1010 is a recombinant, single-chain, unmodified human rIL-12 joined by a flexible ([Gly4Ser]5) linker to the FHAB domain, (see for example Richard T Kenney et al. Front Immunol. 2024 Feb 29:15:1362775).

XTX-301 is a protein-engineered IL-12 prodrug that is masked with a protein domain to block binding activity until IL-12 is cleaved by TME-related proteases (see for example Ekta Patel ImmunoTher Cancer (2021) 9(Suppl 2):A748-8).

ANK-101 is composed of a single-chain human IL-12 protein fused to a C-terminal, phosphorylated ABP (IL-12-ABP) complexed with Alhydrogel (see for example Sailaja Battula et al. JCI Insight. 2023 Dec 8; 8(23): e168224).

CLN-617 is a single-chain fusion protein comprised of human IL-2, leukocyte-associated immunoglobulin-like receptor 2 (LAIR2), human serum albumin (HSA), and human IL-12 (Naveen K. Mehta, Cancer Res (2023) 83 (7_Supplement): 1839).

WTX-330 is a systemically administered, selectively activated IL-12 prodrug consisting of an HSA to extend its half-life and wild-type IL-12 masked with a high-affinity anti-IL-12 scFv that are connected via tumor protease-sensitive linkers (see for example Christopher J Nirschl et al., Cancer Immunol Res. 2023 Jul 5;11(7):962-977).

XmAb-662 is a potency-reduced IL12-Fc fusion protein (see for example Yong Fu et al. Front Immunol. 2023; 14: 1218082. Doi: 10.3389/fimmu.2023.1218082).

PD1-IL12 is designed to circulate in an inactive form, thereby reducing IL-12-drived toxicity. Upon binding to PD-1 on a tumor-reactive T cell, PD-1-dependent IL-12 undergoes a conformational change to reveal active IL-12, which can then signal in cis. In addition to selectively delivering IL-12, PD-1-dependent IL-12 functionally blocks PD-1/PD-L1 signaling.

ATP-IL12 corresponds to ATP-dependent IL-12 engineered to remain inactive in circulation to avoid the systemic toxicity associated with recombinant IL-12 therapy. Following binding to extracellular ATP present in the tumor, ATP-dependent IL-12 undergoes a conformational change, and the active IL-12 component becomes accessible to IL-12R. IL-12 signaling then drives anti-cancer immunity though effector immune cell differentiation and induction of IFN-γ production.

Split-IL-12 is obtained by splitting IL-12 into two inactive components which are each targeted directly to the tumor microenvironment using a tumor-specific antibody. Each inactive subunit is administered separately so that they fuse and become functionally active only at the site of the tumor, avoiding activation in the bloodstream, where IL-12 can cause off-target toxicities (see Joshua Heiber et al. Cancer Res (2024) 84 (6_Supplement): 4066).

InC-01 (InCephalo's Compartment Locked IL-12 cytokine) is a locally applied biological drug candidate designed for the treatment of cancers in the brain.

SON-1210 is a single-chain human IL-12 and a native human IL-15 fused in cis onto a fully human albumin binding (FHAB) domain single-chain antibody fragment (scFv) (see for example John K. Cini et al. Front Immunol. 2023; 14: 1326927. Doi: 10.3389/fimmu.2023.1326927).

ZW-270 is an affinity attenuated, masked protease activated IL-12Fc molecule (see Maya C. Poffenberger et al. Cancer Res (2023) 83 (7_Supplement): 2935).

As previously indicated and defined, such combined administrations may be performed simultaneously, separately, or sequentially in an individual in need thereof, either in the same pharmaceutical composition or in different pharmaceutical compositions according to the invention.

It is also disclosed herein a use of an antibody, or an antigen-binding fragment thereof, or a nucleic acid sequence encoding such antibody or antigen-binding fragment thereof, for the manufacture of a medicament. The medicament may be for the prevention and/or treatment of a cancer in an individual in need thereof.

It is also disclosed herein a method for treating and/or preventing a cancer in an individual in need thereof comprising administering to said subject an effective amount of an antibody, or an antigen-binding fragment thereof, a nucleic acid sequence encoding such antibody or antigen-binding fragment thereof, or a pharmaceutical composition as described herein.

A cancer considered herein may be selected from the group comprising consisting of: Renal Cancer, in particular Renal Cell Carcinoma; Colorectal Cancer; Liver Cancer, in particular Hepatocellular Carcinoma; Lung cancer, in particular Non-Small Cell Lung Cancer (NSCLC); Mesothelioma, in particular Malignant Pleural Mesothelioma; Esophageal Cancer; Head and Neck cancer, in particular Head and Neck Squamous Cell Carcinoma or Cervical Cancer; Urothelial Cancer, in particular Urothelial Carcinoma; Lymphoma, in particular Primary Hodgkin Lymphoma or Large B Cell Lymphoma; Gastric Cancer; Skin cancer, in particular Melanoma, Merkel Cell Carcinoma or Cutaneous Squamous Cell Carcinoma; Endometrial Cancer, in particular Endometrial Carcinoma; Breast Cancer, in particular Triple Negative Breast Cancer or Breast invasive Carcinoma; Pancreas cancer, in particular Pancreatic Adenocarcinoma; Thymoma, Prostate Cancer, in particular Prostate Adenocarcinoma; Ovarian Cancer, in particular Ovarian Serous Cystadenocarcinoma; Thyroid Cancer, in particular Thyroid Carcinoma, Glioblastoma and Sarcoma. It may in particular be selected from the group consisting of Lung cancer, in particular Non-Small Cell Lung Cancer (NSCLC) and Breast Cancer.

The present disclosure is further illustrated, without in any way being limited to, by the Examples hereafter.

### EXAMPLES

### Example 1: Tumor staining experiments

### A. Materials and Methods

### PBMC isolation

Blood pockets containing whole human blood were processed the same day they were received. PBMCs were isolated by Lymphoprep (Stemcell Technologies, ref#04-03-3291/02) gradient centrifugation in 50 mL Blood-Sep-Filter tubes (Dacos, ref#03-7100SI).

After centrifugation (800g, 15 min, no brake) the PBMC layer was removed, and cells were washed with RPMI 1640 (Gibco, ref#61870044) twice.

### Patients' tumor dissociation

Human tumor samples were cut into small pieces and subjected to an enzymatic digestion in CO2 independent medium (Gibco, ref# 18045-088) with DNAse (150 µg/ml) (Roche, 11284932001) and Liberase (75 µg/ml) (Roche, 5401020001) for 30 min at 37°C with agitation.

Samples were filtered (40 µm filter), cells were counted and finally frozen at -150°C in 90% Foetal Calf Serum 10% DMSO until use. Prior to tumor staining, samples were thawed in RPMI 10% at 37°C, washed once and then stained with antibodies for flow cytometry analysis.

### Flow Cytometry Staining

PBMCs or tumor samples were stained with fluorochrome coupled monoclonal antibodies for flow cytometry identification of cell subsets. Particularly, cells were washed twice in PBS 1X, and incubated for 20 min at 4 °C with 1:300 LIVE/DEAD Fixable Aqua Dead Cell Stain Kit (Fischer scientific) and 1:50 human Fc Block (Miltenyi Biotech) in PBS, to exclude dead cells and to block unspecific binding.

After 2 washes in FACS Buffer, cells were stained with antibodies against surface markers: CD45-APC-Cy7, CD3-R718, CD14-BV711, CD56-BV605, CD4-BV7856, CD8-BUV496, CD25-BUV737, CCR8-BUV395 and IL18R1-PE (BD Biosciences). Cells were washed twice, fixed and permeabilized using Foxp3/Transcription Factor Staining Buffer Set (Invitrogen^{™} eBioscience^{™}) following manufacturer's instructions.

Finally, anti-FOXP3-PE-CF594 was used to stain intracellularly the transcription factor that defines Treg cells. Data were collected on a Fortessa X20 flow cytometer (BD Biosciences) and analyzed using FlowJo (Tree star) software.

### B. Tumor staining experiments

The results obtained are represented in **Figure 1****.**

The intensity of expression of IL18R was measured and compared on different immune cells, either in the context of PBMC from healthy donors or in the context of cancers from patients, in particular in the context of two different cancer types from patients: a NSCLC cancer and a breast cancer.

The results obtained demonstrate that IL 18R is differentially expressed by cancer Tregs as compared to Tregs outside of cancers, and in particular is significantly more expressed on the surface of cancer-infiltrating Tregs that on the surface of Tregs outside of a cancer.

### Example 2: Correlation between IL18R positive cells and levels of CD25 and FOXP3 in cancer-infiltrating Tregs

### A. Materials and Methods

The Analysis was performed on the same samples as those defined in Example 1.

### Flow Cytometry Staining

Dissociated tumor samples were washed twice in PBS 1X, and incubated for 20 min at 4 °C with 1:300 LIVE/DEAD Fixable Aqua Dead Cell Stain Kit (Fischer scientific) and 1:50 human Fc Block (Miltenyi Biotech) in PBS, to exclude dead cells and to block unspecific binding.

After 2 washes in FACS Buffer, cells were stained with antibodies against surface markers: CD45-APC-Cy7, CD3-R718, CD14-BV711, CD56-BV605, CD4-BV7856, CD8-BUV496, CD25-BUV737, CCR8-BUV395 and IL18R1-PE (BD Biosciences). Cells were washed twice, fixed and permeabilized using Foxp3/Transcription Factor Staining Buffer Set (Invitrogen^{™} eBioscience^{™}) following manufacturer's instructions.

Finally, anti-FOXP3-PE-CF594 was used to stain intracellularly the transcription factor that defines Treg cells. Data were collected on a Fortessa X20 flow cytometer (BD Biosciences) and analyzed using FlowJo (Tree star) software.

### B. Results

The results obtained are represented in **Figure 2****.**

This study revealed a notable association between II,18R positivity and the expression levels of CD25, FOXP3 and CCR8 in cancer-infiltrating Treg cells. Specifically, it was observed that IL18R positive tumor-infiltrating Treg cells exhibit elevated expression levels of CD25, FOXP3 and CCR8, which are well-established markers associated with Treg suppressive capacity.

These findings suggest a potential correlation between IL18R positivity and enhanced Treg suppressive function within the tumor microenvironment.

Accordingly, not only is IL18R highly expressed in tumor-infiltrating Treg as compared to other Tregs, it is also more specifically and significantly highly expressed in the cancer-infiltrating Tregs associated with stronger suppressive function.

### Example 3: Agonizing activity of antibodies according to the invention

### A. Materials and Methods

### Cell lines

HEK-Blue IL-18^{™} cells (InvivoGen) were used as IL-18 sensor cells. They were maintained in complete media (DMEM, 4.5 g/L glucose, 2mM L-Glutamine, 10% (v/v) heat-inactivated FBS, 100 U/ml penicillin, 100 µg/mL streptomycin, supplemented with 100 µg/mL Normocin and HEK-Blue ^{™} Selection) at 37°C and 5% CO2. HEK-Blue^{™} IL-18 cells have been engineered to detect bioactive IL-18 through the activation of NF-kB and AP-1 pathways.

### HEK-Blue IL-18 activity assay

For antibody functional characterization, 25.000 HEK-Blue^{™} IL-18 cells per well were plated in a 96 well plate flat bottom. The cells were incubated with recombinant human IL-18 (0.8-1800 pg/mL) or with antibodies in a dose response form (0.05-15 µg/mL) for a total volume of 100 µL of test media (DMEM, 4.5 g/L glucose, 2mM L-Glutamine, 10% (v/v) heat-inactivated FBS, 100 U/mL penicillin, 100 µg/mL streptomycin) for 24 hours at 37°C and 5% CO2. Secretory embryonic alkaline phosphatase (SEAP) activity in the supernatant correlates with IL-18R signaling (colorimetric assay: color change from pink to blue), SEAP activity was detected upon combination of 20 µL of cell culture supernatant with 180 µL of Quanti-Blue solution and incubation for 3 hours at 37°C and 5% CO₂. Optical density was measured at 630 nm using a spectrophotometer.

### PBMC IFNγ secretion assay

One million human PBMCs were stimulated in vitro with 1 nM of recombinant human IL-18 (9124-IL-050/CF, R&D) or antibody #3552 ranging from 1-100 µM in a 96-well (low evaporation plates 167574, Thermo Scientific). All stimulations were performed in the presence of recombinant hIL-12 (10 ng/mL, 219-IL/CF, R&D) for 24 hours in 300 µl of AIMV culture media (12055091, Gibco). IFN-γ production after stimulation were measured by CBA (BD Biosciences) per the manufacturers' instructions. As negative control, Ultra-LEAF Purified Human IgG1 Isotype Control (403502, Biolegend) was used.

### B. Results

The results obtained are represented in **Figures 3** **and** **4****.**

Antibody #3552 according to the invention demonstrates an agonist activity on the IL 18R receptor by inducing IL-18 signaling and promotes IFNγ secretion by total PBMCs.

## Claims

1. An isolated agonist anti-IL18R antibody, or a fragment thereof, which selectively inhibits tumor-associated regulatory T cells.

2. The antibody, or fragment thereof, according to claim 1, comprising:
(a) a heavy chain wherein the variable domain comprises:
- a H-CDR1 having at least 88% identity with sequence set forth as SEQ ID NO: 1,
- a H-CDR2 having at least 85% identity with sequence set forth as SEQ ID NO: 2, and
- a H-CDR3 having at least 90% identity with sequence set forth as SEQ ID NO: 3;
and
(b) a light chain wherein the variable domain comprises:
- a L-CDR1 having at least 80% identity with sequence set forth as SEQ ID NO: 5,
- a L-CDR2 having the sequence DTS, and
- a L-CDR3 having at least 88% identity with sequence set forth as SEQ ID NO: 7.

3. The antibody, or fragment thereof, according to claim 1 or 2, comprising (a) a heavy chain wherein the variable domain comprises SEQ ID NO:1 as H-CDR1, SEQ ID NO:2 as H-CDR2 and SEQ ID NO:3 as H-CDR3; and (b) a light chain wherein the variable domain comprises SEQ ID NO:5 as L-CDR1, DTS as L-CDR2 and SEQ ID NO:7 as L-CDR3.

4. The antibody, or fragment thereof, according to any one of claims 1 to 3, comprising:
- a heavy chain variable domain having at least 85 %, in particular at least 90%, identity with SEQ ID NO: 4; and
- a light chain variable domain having at least 85 %, in particular at least 90%, identity with SEQ ID NO: 8.

5. The antibody, or fragment thereof, according to any one of claims 1 to 4, wherein the fragment is selected from the group consisting of Fv, Fab, F(ab')2, Fab', dsFv, scFv, sc(Fv)2 and diabodies.

6. The antibody, or fragment thereof, according to any one of claims 1 to 5, wherein the antibody, or fragment thereof, is a multi-specific antibody, and in particular a bispecific antibody, more particularly a bispecific antibody binding to two different epitopes from IL18R.

7. The antibody, or fragment thereof, according to any one of claims 1 to 6, wherein the antibody, or fragment thereof, is fused to an IL-2 variant which is a regulatory T cell (Treg)-specific IL-2 receptor antagonist.

8. The antibody, or fragment thereof, according to claim 7, wherein the antibody, or fragment thereof, and the IL-2 variant are fused by the intermediate of a linker.

9. An isolated nucleic acid sequence comprising a sequence encoding a heavy chain and/or a light chain of an antibody, or fragment thereof, according to any one of claims 1 to 8.

10. An isolated nucleic acid sequence comprising a sequence encoding an antibody, or antibody fragment thereof, according to any one of claims 1 to 8.

11. A pharmaceutical composition comprising, in a pharmaceutically acceptable carrier, an antibody, or fragment thereof, according to any one of claims 1 to 8.

12. The pharmaceutical composition according to claim 11, or the antibody, or fragment thereof, according to any one of claims 1 to 8, for use in the prevention and/or treatment of cancer in an individual in need thereof.

13. The pharmaceutical composition or the antibody, or fragment thereof, for use according to claim 12, wherein the individual is a human being.

14. The pharmaceutical composition or the antibody, or fragment thereof, for use according to claim 12 or 13, wherein the cancer is selected from the group consisting of: Renal Cancer, in particular Renal Cell Carcinoma; Colorectal Cancer; Liver Cancer, in particular Hepatocellular Carcinoma; Lung cancer, in particular Non-Small Cell Lung Cancer (NSCLC); Mesothelioma, in particular Malignant Pleural Mesothelioma; Esophageal Cancer; Head and Neck cancer, in particular Head and Neck Squamous Cell Carcinoma or Cervical Cancer; Urothelial Cancer, in particular Urothelial Carcinoma; Lymphoma, in particular Primary Hodgkin Lymphoma or Large B Cell Lymphoma; Gastric Cancer; Skin cancer, in particular Melanoma, Merkel Cell Carcinoma or Cutaneous Squamous Cell Carcinoma; Endometrial Cancer, in particular Endometrial Carcinoma; Breast Cancer, in particular Triple Negative Breast Cancer or Breast invasive Carcinoma; Pancreas cancer, in particular Pancreatic Adenocarcinoma; Thymoma, Prostate Cancer, in particular Prostate Adenocarcinoma; Ovarian Cancer, in particular Ovarian Serous Cystadenocarcinoma; Thyroid Cancer, in particular Thyroid Carcinoma, Glioblastoma and Sarcoma.

15. The pharmaceutical composition or the antibody, or fragment thereof, for use according to any one of claims 12 to 14, which is administered to the individual in need thereof in combination with at least one immune check-point inhibitor; preferably anti-PD-1 and/or anti-PDL-1.

16. The pharmaceutical composition or the antibody, or fragment thereof, for use according to any one of claims 12 to 15, which is administered to the individual in need thereof in combination with at least one agonist of the IL-12 pathway in regulatory T cells and in particular in the tumor-associated regulatory T cells, and is in particular administered to the individual in need thereof in combination with at least one agent selected from the group consisting of IL-12; an IL-12 receptor activator, such as a recombinant IL-12 or a drug under development such as DF6002, dodekin, IMNN-001, PDS01ADC, SON-1010, XTX-301, ANK-101, CLN-617, WTX-330, XmAb-662, PD1-IL12, ATP-IL12, split-IL-12, InC-01, SON-1210 and ZW-270.
